# EUROPEAN PATENT SPECIFICATION

(11) **EP 1 599 586 B1**
(45) Date of publication and mention of the grant of the patent: **06.04.2011**
(21) Application number: 04715568.4
(22) Date of filing: 27.02.2004
(51) Int. Cl.: C12N 15/11

(54) **RNA-INTERFERENCE FOR ZNFN3A1-GENE AS A METHOD FOR INHIBITING CANCER CELL GROWTH**
RNA-INTERFERENZ FÜR DAS GEN ZNFN3A1 ALS VERFAHREN ZUR HEMMUNG DES WACHSTUMS VON KREBSZELLEN
INTERFÉRENCE DE L'ARN POUR LE GÈNE ZNFN3A1 COMME MÉTHODE POUR D'INHIBITION DE LA CROISSANCE DE CELLULES CANCEREUSES

(30) Priority: 28.02.2003 US 450644 P
(43) Date of publication of application: 30.11.2005
(60) Divisional application: 10180084.5
(73) Proprietor: Oncotherapy Science, Inc., Kawasaki-shi Kanagawa 213-0012 (JP)
(72) Inventor: NAKAMURA, Yusuke, Yokohama-shi, Kanagawa 225-0011 (JP); FURUKAWA, Yoichi, Kawasaki-shi, Kanagawa216-0033 (JP)
(74) Representative: Vossius & Partner
(86) International application number: PCT/JP2004/002446
(87) International publication number: WO 2004/076623

(56) References cited:
- WO-A-03/010180
- WO-A-03/027143
- HAMAMOTO R ET AL: "ZNFN3A1, A NOVEL GENE THAT PROMOTES CELL GROWTH IN HEPATOCELLULAR CARCINOMA" PROCEEDINGS OF THE ANNUAL MEETING OF THE AMERICAN ASSOCIATION FOR CANCER RESEARCH, NEW YORK, NY, US, vol. 43, no. 13, March 2002 (2002-03), page 13, XP001146350 ISSN: 0197-016X
- ANONYMOUS: "siRNA Design: It's All in the Algorithm" INTERNET ARTICLE, [Online] 2004, pages 1-4, XP002299086 Retrieved from the Internet: URL:HTTP://WWW.AMBION.COM/TECHLIB/TN/113/1 4.HTML> [retrieved on 2004-10-03] cited in the application

### SEQUENCE LISTING

<110> ONCOTHERAPY SCIENCE, INC.
   JAPAN AS REPRESENTED BY THE PRESIDENT OF THE UNIVERSITY OF TOKYO
<120> COMPOSITIONS AND METHODS OF INHIBITING CELL GROWTH
<130> ONC-A0301P
<150> US 60/450,644
   <151> 2003-02-28
<160> 69
<170> PatentIn version 3.1
<210> 1
   <211> 1622
   <212> DNA
   <213> Homo sapiens
<220>
   <221> CDS
   <222> (96)..(1382)
   <223>
<400> 1
   gtgcgcgcag ggcgcaggcg cgcgggtccc ggcagcccgt gagacgcccg ctgctggacg        60
   cgggtagccg tctgaggtgc cggagctgcg ggagg atg gag ccg ctg aag gtg          113
                                          Met Glu Pro Leu Lys Val
                                          1               5
   gaa aag ttc gca acc gcc aac agg gga aac ggg ctg cgc gcc gtg acc          161
   Glu Lys Phe Ala Thr Ala Asn Arg Gly Asn Gly Leu Arg Ala Val Thr
               10                  15                  20
   ccg ctg cgc ccc gga gag cta ctc ttc cgc tcg gat ccc ttg gcg tac          209
   Pro Leu Arg Pro Gly Glu Leu Leu Phe Arg Ser Asp Pro Leu Ala Tyr
           25                  30                  35
   acg gtg tgc aag ggg agt cgt ggc gtc gtc tgc gac cgc tgc ctt ctc          257
   Thr Val Cys Lys Gly Ser Arg Gly Val Val Cys Asp Arg Cys Leu Leu
   40 45 50
   ggg aag gaa aag ctg atg cga tgc tct cag tgc cgc gtc gcc aaa tac 305
   Gly Lys Glu Lys Leu Met Arg Cys Ser Gln Cys Arg Val Ala Lys Tyr
   55 60 65 70
   tgt agt gct aag tgt cag aaa aaa gct tgg cca gac cac aag cgg gaa 353
   Cys Ser Ala Lys Cys Gln Lys Lys Ala Trp Pro Asp His Lys Arg Glu
   75 80 85
   tgc aaa tgc ctt aaa agc tgc aaa ccc aga tat cct cca gac tcc gtt 401
   Cys Lys Cys Leu Lys Ser Cys Lys Pro Arg Tyr Pro Pro Asp Ser Val
   90 95 100
   cga ctt ctt ggc aga gtt gtc ttc aaa ctt atg gat gga gca cct tca 449
   Arg Leu Leu Gly Arg Val Val Phe Lys Leu Met Asp Gly Ala Pro Ser
   105 110 115
   gaa tca gag aag ctt tac tca ttt tat gat ctg gag tca aat att aac 497
   Glu Ser Glu Lys Leu Tyr Ser Phe Tyr Asp Leu Glu Ser Asn Ile Asn
   120 125 130
   aaa ctg act gaa gat aag aaa gag ggc ctc agg caa ctc gta atg aca 545
   Lys Leu Thr Glu Asp Lys Lys Glu Gly Leu Arg Gln Leu Val Met Thr
   135 140 145 150
   ttt caa cat ttc atg aga gaa gaa ata cag gat gcc tct cag ctg cca 593
   Phe Gln His Phe Met Arg Glu Glu Ile Gln Asp Ala Ser Gln Leu Pro
   155 160 165
   cct gcc ttt gac ctt ttt gaa gcc ttt gca aaa gtg atc tgc aac tct 641
   Pro Ala Phe Asp Leu Phe Glu Ala Phe Ala Lys Val Ile Cys Asn Ser
   170 175 180
   ttc acc atc tgt aat gcg gag atg cag gaa gtt ggt gtt ggc cta tat 689
   Phe Thr Ile Cys Asn Ala Glu Met Gln Glu Val Gly Val Gly Leu Tyr
   185 190 195
   ccc agt atc tct ttg ctc aat cac agc tgt gac ccc aac tgt tcg att 737
   Pro Ser Ile Ser Leu Leu Asn His Ser Cys Asp Pro Asn Cys Ser Ile
   200 205 210
   gtg ttc aat ggg ccc cac ctc tta ctg cga gca gtc cga gac atc gag 785
   Val Phe Asn Gly Pro His Leu Leu Leu Arg Ala Val Arg Asp Ile Glu
   215 220 225 230
   gtg gga gag gag ctc acc atc tgc tac ctg gat atg ctg atg acc agt 833
   Val Gly Glu Glu Leu Thr Ile Cys Tyr Leu Asp Met Leu Met Thr Ser
   235 240 245
   gag gag cgc cgg aag cag ctg agg gac cag tac tgc ttt gaa tgt gac 881
   Glu Glu Arg Arg Lys Gln Leu Arg Asp Gln Tyr Cys Phe Glu Cys Asp
   250 255 260
   tgt ttc cgt tgc caa acc cag gac aag gat gct gat atg cta act ggt 929
   Cys Phe Arg Cys Gln Thr Gln Asp Lys Asp Ala Asp Met Leu Thr Gly
   265 270 275
   gat gag caa gta tgg aag gaa gtt caa gaa tcc ctg aaa aaa att gaa 977
   Asp Glu Gln Val Trp Lys Glu Val Gln Glu Ser Leu Lys Lys Ile Glu
   280 285 290
   gaa ctg aag gca cac tgg aag tgg gag cag gtt ctg gcc atg tgc cag 1025
   Glu Leu Lys Ala His Trp Lys Trp Glu Gln Val Leu Ala Met Cys Gln
   295 300 305 310
   gcg atc ata agc agc aat tct gaa cgg ctt ccc gat atc aac atc tac 1073
   Ala Ile Ile Ser Ser Asn Ser Glu Arg Leu Pro Asp Ile Asn Ile Tyr
   315 320 325
   cag ctg aag gtg ctc gac tgc gcc atg gat gcc tgc atc aac ctc ggc 1121
   Gln Leu Lys Val Leu Asp Cys Ala Met Asp Ala Cys Ile Asn Leu Gly
   330 335 340
   ctg ttg gag gaa gcc ttg ttc tat ggt act cgg acc atg gag cca tac 1169
   Leu Leu Glu Glu Ala Leu Phe Tyr Gly Thr Arg Thr Met Glu Pro Tyr
   345 350 355
   agg att ttt ttc cca gga agc cat ccc gtc aga ggg gtt caa gtg atg 1217
   Arg Ile Phe Phe Pro Gly Ser His Pro Val Arg Gly Val Gln Val Met
   360 365 370
   aaa gtt ggc aaa ctg cag cta cat caa ggc atg ttt ccc caa gca atg 1265
   Lys Val Gly Lys Leu Gln Leu His Gln Gly Met Phe Pro Gln Ala Met
   375 380 385 390
   aag aat ctg aga ctg gct ttt gat att atg aga gtg aca cat ggc aga      1313
   Lys Asn Leu Arg Leu Ala Phe Asp Ile Met Arg Val Thr His Gly Arg
                   395                     400                     405
   gaa cac agc ctg att gaa gat ttg att cta ctt tta gaa gaa tgc gac      1361
   Glu His Ser Leu Ile Glu Asp Leu Ile Leu Leu Leu Glu Glu Cys Asp
               410                     415                     420
   gcc aac atc aga gca tcc taa gggaacgcag tcagagggaa atacggcgtg         1412
   Ala Asn Ile Arg Ala Ser
           425
   tgtctttgtt gaatgcctta ttgaggtcac acactctatg ctttgttagc tgtgtgaacc    1472
   tctcttattg gaaattctgt tccgtgtttg tgtaggtaaa taaaggcaga catggtttgc    1532
   aaaccacaag aatcattagt tgtagagaag cacgattata ataaattcaa aacatttggt    1592
   tgaggatgcc aaaaaaaaaa aaaaaaaaaa                                     1622
<210> 2
   <211> 428
   <212> PRT
   <213> Homo sapiens
<400> 2
   Met Glu Pro Leu Lys Val Glu Lys Phe Ala Thr Ala Asn Arg Gly Asn
   1               5                   10                  15
   Gly Leu Arg Ala Val Thr Pro Leu Arg Pro Gly Glu Leu Leu Phe Arg
               20                  25                  30
   Ser Asp Pro Leu Ala Tyr Thr Val Cys Lys Gly Ser Arg Gly Val Val
           35                  40                  45
   Cys Asp Arg Cys Leu Leu Gly Lys Glu Lys Leu Met Arg Cys Ser Gln
       50                  55                  60
   Cys Arg Val Ala Lys Tyr Cys Ser Ala Lys Cys Gln Lys Lys Ala Trp
   65                  70                  75                  80
   Pro Asp His Lys Arg Glu Cys Lys Cys Leu Lys Ser Cys Lys Pro Arg
                   85                  90                  95
   Tyr Pro Pro Asp Ser Val Arg Leu Leu Gly Arg Val Val Phe Lys Leu
   100 105 110
   Met Asp Gly Ala Pro Ser Glu Ser Glu Lys Leu Tyr Ser Phe Tyr Asp
   115 120 125
   Leu Glu Ser Asn Ile Asn Lys Leu Thr Glu Asp Lys Lys Glu Gly Leu
   130 135 140
   Arg Gln Leu Val Met Thr Phe Gln His Phe Met Arg Glu Glu Ile Gln
   145 150 155 160
   Asp Ala Ser Gln Leu Pro Pro Ala Phe Asp Leu Phe Glu Ala Phe Ala
   165 170 175
   Lys Val Ile Cys Asn Ser Phe Thr Ile Cys Asn Ala Glu Met Gln Glu
   180 185 190
   Val Gly Val Gly Leu Tyr Pro Ser Ile Ser Leu Leu Asn His Ser Cys
   195 200 205
   Asp Pro Asn Cys Ser Ile Val Phe Asn Gly Pro His Leu Leu Leu Arg
   210 215 220
   Ala Val Arg Asp Ile Glu Val Gly Glu Glu Leu Thr Ile Cys Tyr Leu
   225 230 235 240
   Asp Met Leu Met Thr Ser Glu Glu Arg Arg Lys Gln Leu Arg Asp Gln
   245 250 255
   Tyr Cys Phe Glu Cys Asp Cys Phe Arg Cys Gln Thr Gln Asp Lys Asp
   260 265 270
   Ala Asp Met Leu Thr Gly Asp Glu Gln Val Trp Lys Glu Val Gln Glu
   275 280 285
   Ser Leu Lys Lys Ile Glu Glu Leu Lys Ala His Trp Lys Trp Glu Gln
   290 295 300
   Val Leu Ala Met Cys Gln Ala Ile Ile Ser Ser Asn Ser Glu Arg Leu
   305 310 315 320
   Pro Asp Ile Asn Ile Tyr Gln Leu Lys Val Leu Asp Cys Ala Met Asp
   325 330 335
   Ala Cys Ile Asn Leu Gly Leu Leu Glu Glu Ala Leu Phe Tyr Gly Thr
   340 345 350
   Arg Thr Met Glu Pro Tyr Arg Ile Phe Phe Pro Gly Ser His Pro Val
   355 360 365
   Arg Gly Val Gln Val Met Lys Val Gly Lys Leu Gln Leu His Gln Gly
   370 375 380
   Met Phe Pro Gln Ala Met Lys Asn Leu Arg Leu Ala Phe Asp Ile Met
   385 390 395 400
   Arg Val Thr His Gly Arg Glu His Ser Leu Ile Glu Asp Leu Ile Leu
   405 410 415
   Leu Leu Glu Glu Cys Asp Ala Asn Ile Arg Ala Ser
   420 425
<210> 3
   <211> 55
   <212> DNA
   <213> Artificial
<220>
   <223> An artificially synthesized origonucleotide sequence for siRNA
<400> 3
   caccaaactt atggatggag cacctttcaa gagaaggtgc tccatccata agttt 55
<210> 4
   <211> 55
   <212> DNA
   <213> Artificial
<220>
   <223> An artificially synthesized origonucleotide sequence for siRNA
<400> 4
   aaaaaaactt atggatggag caccttctct tgaaaggtgc tccatccata agttt 55
<210> 5
   <211> 55
   <212> DNA
   <213> Artificial
<220>
   <223> An artificially synthesized origonucleotide sequence for siRNA
<400> 5
   caccaatcag agaagcttta ctcatttcaa gagaatgagt aaagcttctc tgatt 55
<210> 6
   <211> 55
   <212> DNA
   <213>' Artificial
<220>
   <223> An artificially synthesized origonucleotide sequence for siRNA
<400> 6
   aaaaaatcag agaagcttta ctcattctct tgaaatgagt aaagcttctc tgatt 55
<210> 7
   <211> 55
   <212> DNA
   <213> Artificial
<220>
   <223> An artificially synthesized origonucleotide sequence for siRNA
<400> 7
   caccaacaaa ctgactgaag ataagttcaa gagacttatc ttcagtcagt ttgtt 55
<210> 8
   <211> 55
   <212> DNA
   <213> Artificial
<220>
   <223> An artificially synthesized origonucleotide sequence for siRNA
<400> 8
   aaaaaacaaa ctgactgaag ataagtctct tgaacttatc ttcagtcagt ttgtt 55
<210> 9
   <211> 55
   <212> DNA
   <213> Artificial
<220>
   <223> An artificially synthesized origonucleotide sequence for siRNA
<400> 9
   caccaactcg taatgacatt tcaacttcaa gagagttgaa atgtcattac gagtt 55
<210> 10
   <211> 55
   <212> DNA
   <213> Artificial
<220>
   <223> An artificially synthesized origonucleotide sequence for siRNA
<400> 10
   aaaaaactcg taatgacatt tcaactctct tgaagttgaa atgtcattac gagtt 55
<210> 11
   <211> 55
   <212> DNA
   <213> Artificial
<220>
   <223> An artificially synthesized origonucleotide sequence for siRNA
<400> 11
   caccaaaagt gatctgcaac tctttttcaa gagaaaagag ttgcagatca ctttt 55
<210> 12
   <211> 55
   <212> DNA
   <213> Artificial
<220>
   <223> An artificially synthesized origonucleotide sequence for siRNA
<400> 12
   aaaaaaaagt gatctgcaac tcttttctct tgaaaaagag ttgcagatca ctttt 55
<210> 13
   <211> 55
   <212> DNA
   <213> Artificial
<220>
   <223> An artificially synthesized origonucleotide sequence for siRNA
<400> 13
   caccaagtga tctgcaactc tttcattcaa gagatgaaag agttgcagat cactt 55
<210> 14
   <211> 55
   <212> DNA
   <213> Artificial
<220>
   <223> An artificially synthesized origonucleotide sequence for siRNA
<400> 14
   aaaaaagtga tctgcaactc tttcatctct tgaatgaaag agttgcagat cactt 55
<210> 15
   <211> 55
   <212> DNA
   <213> Artificial
<220>
   <223> An artificially synthesized origonucleotide sequence for siRNA
<400> 15
   caccaactct ttcaccatct gtaatttcaa gagaattaca gatggtgaaa gagtt 55
<210> 16
   <211> 55
   <212> DNA
   <213> Artificial
<220>
   <223> An artificially synthesized origonucleotide sequence for siRNA
<400> 16
   aaaaaactct ttcaccatct gtaattctct tgaaattaca gatggtgaaa gagtt 55
<210> 17
   <211> 55
   <212> DNA
   <213> Artificial
<220>
   <223> An artificially synthesized origonucleotide sequence for siRNA
<400> 17
   caccaactgt tcgattgtgt tcaatttcaa gagaattgaa cacaatcgaa cagtt 55
<210> 18
   <211> 55
   <212> DNA
   <213> Artificial
<220>
   <223> An artificially synthesized origonucleotide sequence for siRNA
<400> 18
   aaaaaactgt tcgattgtgt tcaattctct tgaaattgaa cacaatcgaa cagtt 55
<210> 19
   <211> 55
   <212> DNA
<213> Artificial
<220>
   <223> An artificially synthesized origonucleotide sequence for siRNA
<400> 19
   caccaaggat gctgatatgc taactttcaa gagaagttag catatcagca tcctt 55
<210> 20
   <211> 55
   <212> DNA
   <213> Artificial
<220>
   <223> An artificially synthesized origonucleotide sequence for siRNA
<400> 20
   aaaaaaggat gctgatatgc taacttctct tgaaagttag catatcagca tcctt 55
<210> 21
   <211> 55
   <212> DNA
   <213> Artificial
<220>
   <223> An artificially synthesized origonucleotide sequence for siRNA
<400> 21
   caccaactgg tgatgagcaa gtatgttcaa gagacatact tgctcatcac cagtt 55
<210> 22
   <211> 55
   <212> DNA
   <213> Artificial
<220>
   <223> An artificially synthesized origonucleotide sequence for siRNA
<400> 22
   aaaaaactgg tgatgagcaa gtatgtctct tgaacatact tgctcatcac cagtt 55
<210> 23
   <211> 55
   <212> DNA
   <213> Artificial
<220>
   <223> An artificially synthesized origonucleotide sequence for siRNA
<400> 23
   caccaagtat ggaaggaagt tcaagttcaa gagacttgaa cttccttcca tactt 55
<210> 24
   <211> 55
   <212> DNA
   <213> Artificial
<220>
   <223> An artificially synthesized origonucleotide sequence for siRNA
<400> 24
   aaaaaagtat ggaaggaagt tcaagtctct tgaacttgaa cttccttcca tactt 55
<210> 25
   <211> 55
   <212> DNA
   <213> Artificial
<220>
   <223> An artificially synthesized origonucleotide sequence for siRNA
<400> 25
   caccaacatc taccagctga aggtgttcaa gagacacctt cagctggtag atgtt 55
<210> 26
   <211> 55
   <212> DNA
   <213> Artificial
<220>
   <223> An artificially synthesized origonucleotide sequence for siRNA
<400> 26
   aaaaaacatc taccagctga aggtgtctct tgaacacctt cagctggtag atgtt 55
<210> 27
   <211> 55
   <212> DNA
   <213> Artificial
<220>
   <223> An artificially synthesized origonucleotide sequence for siRNA
<400> 27
   caccaagcaa tgaagaatct gagacttcaa gagagtctca gattcttcat tgctt 55
<210> 28
   <211> 55
   <212> DNA
   <213> Artificial
<220>
   <223> An artificially synthesized origonucleotide sequence for siRNA
<400> 28
   aaaaaagcaa tgaagaatct gagactctct tgaagtctca gattcttcat tgctt 55
<210> 29
   <211> 22
   <212> DNA
   <213> Artificial
<220>
   <223> An artificially synthesized primer sequence
<400> 29
   acaacagcct caagatcatc ag 22
<210> 30
   <211> 20
   <212> DNA
   <213> Artificial
<220>
   <223> An artificially synthesized primer sequence
<400> 30
   ggtccaccac tgacacgttg 20
<210> 31
   <211> 23
   <212> DNA
   <213> Artificial
<220>
   <223> An artificially synthesized primer sequence
<400> 31
   ttcccgatat caacatctac cag 23
<210> 32
   <211> 23
   <212> DNA
   <213> Artificial
<220>
   <223> An artificially synthesized primer sequence
<400> 32
   agtgtgtgac ctcaataagg cat 23
<210> 33
   <211> 4869
   <212> DNA
   <213> Artificial
<220>
   <223> An artificially synthesized plasmid sequence
<220>
   <221> misc_feature
   <222> (485).. (490)
   <223> n indicates GAP.
<400> 33
   gacggatcgg gagatctccc gatcccctat ggtgcactct cagtacaatc tgctctggat 60
   ccactagtaa cggccgccag tgtgctggaa ttcggcttgg ggatcagcgt ttgagtaaga 120
   gcccgcgtct gaaccctccg cgccgccccg gccccagtgg aaagacgcgc aggcaaaacg 180
   caccacgtga cggagcgtga ccgcgcgccg agcgcgcgcc aaggtcgggc aggaagaggg 240
   cctatttccc atgattcctt catatttgca tatacgatac aaggctgtta gagagataat 300
   tagaattaat ttgactgtaa acacaaagat attagtacaa aatacgtgac gtagaaagta 360
   ataatttctt gggtagtttg cagttttaaa attatgtttt aaaatggact atcatatgct 420
   taccgtaact tgaaagtatt tcgatttctt ggctttatat atcttgtgga aaggacgaaa 480
   caccnnnnnn tttttacatc aggttgtttt tctgtttggt ttttttttta caccacgttt 540
   atacgccggt gcacggttta ccactgaaaa cacctttcat ctacaggtga tatcttttaa 600
   cacaaataaa atgtagtagt cctaggagac ggaatagaag gaggtggggc ctaaagccga 660
   attctgcaga tatccatcac actggcggcc gctcgagtga ggcggaaaga accagctggg 720
   gctctagggg gtatccccac gcgccctgta gcggcgcatt aagcgcggcg ggtgtggtgg 780
   ttacgcgcag cgtgaccgct acacttgcca gcgccctagc gcccgctcct ttcgctttct 840
   tcccttcctt tctcgccacg ttcgccggct ttccccgtca agctctaaat cgggggctcc 900
   ctttagggtt ccgatttagt gctttacggc acctcgaccc caaaaaactt gattagggtg 960
   atggttcacg tagtgggcca tcgccctgat agacggtttt tcgccctttg acgttggagt 1020
   ccacgttctt taatagtgga ctcttgttcc aaactggaac aacactcaac cctatctcgg 1080
   tctattcttt tgatttataa gggattttgc cgatttcggc ctattggtta aaaaatgagc 1140
   tgatttaaca aaaatttaac gcgaattaat tctgtggaat gtgtgtcagt tagggtgtgg 1200
   aaagtcccca ggctccccag caggcagaag tatgcaaagc atgcatctca attagtcagc 1260
   aaccaggtgt ggaaagtccc caggctcccc agcaggcaga agtatgcaaa gcatgcatct 1320
   caattagtca gcaaccatag tcccgcccct aactccgccc atcccgcccc taactccgcc 1380
   cagttccgcc cattctccgc cccatggctg actaattttt tttatttatg cagaggccga 1440
   ggccgcctct gcctctgagc tattccagaa gtagtgagga ggcttttttg gaggcctagg 1500
   cttttgcaaa aagctcccgg gagcttgtat atccattttc ggatctgatc aagagacagg 1560
   atgaggatcg tttcgcatga ttgaacaaga tggattgcac gcaggttctc cggccgcttg 1620
   ggtggagagg ctattcggct atgactgggc acaacagaca atcggctgct ctgatgccgc 1680
   cgtgttccgg ctgtcagcgc aggggcgccc ggttcttttt gtcaagaccg acctgtccgg 1740
   tgccctgaat gaactgcagg acgaggcagc gcggctatcg tggctggcca cgacgggcgt 1800
   tccttgcgca gctgtgctcg acgttgtcac tgaagcggga agggactggc tgctattggg 1860
   cgaagtgccg gggcaggatc tcctgtcatc tcaccttgct cctgccgaga aagtatccat 1920
   catggctgat gcaatgcggc ggctgcatac gcttgatccg gctacctgcc cattcgacca 1980
   ccaagcgaaa catcgcatcg agcgagcacg tactcggatg gaagccggtc ttgtcgatca 2040
   ggatgatctg gacgaagagc atcaggggct cgcgccagcc gaactgttcg ccaggctcaa 2100
   ggcgcgcatg cccgacggcg aggatctcgt cgtgacccat ggcgatgcct gcttgccgaa 2160
   tatcatggtg gaaaatggcc gcttttctgg attcatcgac tgtggccggc tgggtgtggc 2220
   ggaccgctat caggacatag cgttggctac ccgtgatatt gctgaagagc ttggcggcga 2280
   atgggctgac cgcttcctcg tgctttacgg tatcgccgct cccgattcgc agcgcatcgc 2340
   cttctatcgc cttcttgacg agttcttctg agcgggactc tggggttcga aatgaccgac 2400
   caagcgacgc ccaacctgcc atcacgagat ttcgattcca ccgccgcctt ctatgaaagg 2460
   ttgggcttcg gaatcgtttt ccgggacgcc ggctggatga tcctccagcg cggggatctc 2520
   atgctggagt tcttcgccca ccccaacttg tttattgcag cttataatgg ttacaaataa 2580
   agcaatagca tcacaaattt cacaaataaa gcattttttt cactgcattc tagttgtggt 2640
   ttgtccaaac tcatcaatgt atcttatcat gtctgtatac cgtcgacctc tagctagagc 2700
   ttggcgtaat catggtcata gctgtttcct gtgtgaaatt gttatccgct cacaattcca 2760
   cacaacatac gagccggaag cataaagtgt aaagcctggg gtgcctaatg agtgagctaa 2820
   ctcacattaa ttgcgttgcg ctcactgccc gctttccagt cgggaaacct gtcgtgccag 2880
   ctgcattaat gaatcggcca acgcgcgggg agaggcggtt tgcgtattgg gcgctcttcc 2940
   gcttcctcgc tcactgactc gctgcgctcg gtcgttcggc tgcggcgagc ggtatcagct 3000
   cactcaaagg cggtaatacg gttatccaca gaatcagggg ataacgcagg aaagaacatg 3060
   tgagcaaaag gccagcaaaa ggccaggaac cgtaaaaagg ccgcgttgct ggcgtttttc 3120
   cataggctcc gcccccctga cgagcatcac aaaaatcgac gctcaagtca gaggtggcga 3180
   aacccgacag gactataaag ataccaggcg tttccccctg gaagctccct cgtgcgctct 3240
   cctgttccga ccctgccgct taccggatac ctgtccgcct ttctcccttc gggaagcgtg 3300
   gcgctttctc atagctcacg ctgtaggtat ctcagttcgg tgtaggtcgt tcgctccaag 3360
   ctgggctgtg tgcacgaacc ccccgttcag cccgaccgct gcgccttatc cggtaactat 3420
   cgtcttgagt ccaacccggt aagacacgac ttatcgccac tggcagcagc cactggtaac 3480
   aggattagca gagcgaggta tgtaggcggt gctacagagt tcttgaagtg gtggcctaac 3540
   tacggctaca ctagaagaac agtatttggt atctgcgctc tgctgaagcc agttaccttc 3600
   ggaaaaagag ttggtagctc ttgatccggc aaacaaacca ccgctggtag cggttttttt 3660
   gtttgcaagc agcagattac gcgcagaaaa aaaggatctc aagaagatcc tttgatcttt 3720
   tctacggggt ctgacgctca gtggaacgaa aactcacgtt aagggatttt ggtcatgaga 3780
   ttatcaaaaa ggatcttcac ctagatcctt ttaaattaaa aatgaagttt taaatcaatc 3840
   taaagtatat atgagtaaac ttggtctgac agttaccaat gcttaatcag tgaggcacct 3900
   atctcagcga tctgtctatt tcgttcatcc atagttgcct gactccccgt cgtgtagata 3960
   actacgatac gggagggctt accatctggc cccagtgctg caatgatacc gcgagaccca 4020
   cgctcaccgg ctccagattt atcagcaata aaccagccag ccggaagggc cgagcgcaga 4080
   agtggtcctg caactttatc cgcctccatc cagtctatta attgttgccg ggaagctaga 4140
   gtaagtagtt cgccagttaa tagtttgcgc aacgttgttg ccattgctac aggcatcgtg 4200
   gtgtcacgct cgtcgtttgg tatggcttca ttcagctccg gttcccaacg atcaaggcga 4260
   gttacatgat cccccatgtt gtgcaaaaaa gcggttagct ccttcggtcc tccgatcgtt 4320
   gtcagaagta agttggccgc agtgttatca ctcatggtta tggcagcact gcataattct 4380
   cttactgtca tgccatccgt aagatgcttt tctgtgactg gtgagtactc aaccaagtca 4440
   ttctgagaat agtgtatgcg gcgaccgagt tgctcttgcc cggcgtcaat acgggataat 4500
   accgcgccac atagcagaac tttaaaagtg ctcatcattg gaaaacgttc ttcggggcga 4560
   aaactctcaa ggatcttacc gctgttgaga tccagttcga tgtaacccac tcgtgcaccc 4620
   aactgatctt cagcatcttt tactttcacc agcgtttctg ggtgagcaaa aacaggaagg 4680
   caaaatgccg caaaaaaggg aataagggcg acacggaaat gttgaatact catactcttc 4740
   ctttttcaat attattgaag catttatcag ggttattgtc tcatgagcgg atacatattt 4800
   gaatgtattt agaaaaataa acaaataggg gttccgcgca catttccccg aaaagtgcca 4860
   cctgacgtc 4869
<210> 34
   <211> 20
   <212> DNA
   <213> Artificial
<220>
   <223> An artificially synthesized primer sequence
<400> 34
   ggggatcagc gtttgagtaa 20
<210> 35
   <211> 20
   <212> DNA
   <213> Artificial
<220>
   <223> An artificially synthesized primer sequence
<400> 35
   taggccccac ctccttctat 20
<210> 36
   <211> 30
   <212> DNA
   <213> Artificial
<220>
   <223> An artificially synthesized primer sequence
<400> 36
   tgcggatcca gagcagattg tactgagagt 30
<210> 37
   <211> 29
   <212> DNA
   <213> Artificial
<220>
   <223> An artificially synthesized primer sequence
<400> 37
   ctctatctcg agtgaggcgg aaagaacca 29
<210> 38
   <211> 40
   <212> DNA
   <213> Artificial
<220>
   <223> An artificially synthesized primer sequence
<400> 38
   tttaagcttg aagactattt ttacatcagg ttgtttttct 40
<210> 39
   <211> 37
   <212> DNA
   <213> Artificial
<220>
   <223> An artificially synthesized primer sequence
<400> 39
   tttaagcttg aagacacggt gtttcgtcct ttccaca 37
<210> 40
   <211> 51
   <212> DNA
   <213> Artificial
<220>
   <223> An artificially synthesized double-stranded oligonucleotide seque nce
<400> 40
   caccgaagca gcacgacttc ttcttcaaga gagaagaagt cgtgctgctt c 51
<210> 41
   <211> 51
   <212> DNA
   <213> Artificial
<220>
   <223> An artificially synthesized double-stranded oligonucleotide seque nce
<400> 41
   aaaagaagca gcacgacttc ttctctcttg aagaagaagt cgtgctgctt c 51
<210> 42
   <211> 30
   <212> DNA
   <213> Artificial
<220>
   <223> An artificially synthesized primer sequence
<400> 42
   ggggtaccag gatggagccg ctgaaggtgg 30
<210> 43
   <211> 33
   <212> DNA
   <213> Artificial
<220>
   <223> An artificially synthesized primer sequence
<400> 43
   gggaattctt aggatgctct gatgttggcg tcg 33
<210> 44
   <211> 51
   <212> DNA
   <213> Artificial
<220>
   <223> An artificially synthesized hairpin siRNA sequence
<400> 44
   aaacttatgg atggagcacc tttcaagaga aggtgctcca tccataagtt t 51
<210> 45
   <211> 51
   <212> DNA
   <213> Artificial
<220>
   <223> An artificially synthesized hairpin siRNA sequence
<400> 45
   aatcagagaa gctttactca tttcaagaga atgagtaaag cttctctgat t 51
<210> 46
   <211> 51
   <212> DNA
   <213> Artificial
<220>
   <223> An artificially synthesized hairpin siRNA sequence
<400> 46
   aacaaactga ctgaagataa gttcaagaga cttatcttca gtcagtttgt t 51
<210> 47
   <211> 51
   <212> DNA
   <213> Artificial
<220>
   <223> An artificially synthesized hairpin siRNA sequence
<400> 47
   aactcgtaat gacatttcaa cttcaagaga gttgaaatgt cattacgagt t 51
<214> 48
   <211> 51
   <212> DNA
   <213> Artificial
<220>
   <223> An artificially synthesized hairpin siRNA sequence
<400> 48
   aaaagtgatc tgcaactctt tttcaagaga aaagagttgc agatcacttt t 51
<210> 49
   <211> 51
   <212> DNA
   <213> Artificial
<220>
   <223> An artificially synthesized hairpin siRNA sequence
<400> 49
   aagtgatctg caactctttc attcaagaga tgaaagagtt gcagatcact t 51
<210> 50
   <211> 51
   <212> DNA
   <213> Artificial
<220>
   <223> An artificially synthesized hairpin siRNA sequence
<400> 50
   aactctttca ccatctgtaa tttcaagaga attacagatg gtgaaagagt t 51
<210> 51
   <211> 51
   <212> DNA
   <213> Artificial
<220>
   <223> An artificially synthesized hairpin siRNA sequence
<400> 51
   aactgttcga ttgtgttcaa tttcaagaga attgaacaca atcgaacagt t 51
<210> 52
   <211> 51
   <212> DNA
   <213> Artificial
<220>
   <223> An artificially synthesized hairpin siRNA sequence
<400> 52
   aaggatgctg atatgctaac tttcaagaga agttagcata tcagcatcct t 51
<210> 53
   <211> 51
   <212> DNA
   <213> Artificial
<220>
   <223> An artificially synthesized hairpin siRNA sequence
<400> 53
   aactggtgat gagcaagtat gttcaagaga catacttgct catcaccagt t 51
<210> 54
   <211> 51
   <212> DNA
   <213> Artificial
<220>
   <223> An artificially synthesized hairpin siRNA sequence
<400> 54
   aagtatggaa ggaagttcaa gttcaagaga cttgaacttc cttccatact t 51
<210> 55
   <211> 51
   <212> DNA
   <213> Artificial
<220>
   <223> An artificially synthesized hairpin siRNA sequence
<400> 55
   aacatctacc agctgaaggt gttcaagaga caccttcagc tggtagatgt t 51
<210> 56
   <211> 51
   <212> DNA
   <213> Artificial
<220>
   <223> An artificially synthesized hairpin siRNA sequence
<400> 56
   aagcaatgaa gaatctgaga cttcaagaga gtctcagatt cttcattgct t 51
<210> 57
   <211> 21
   <212> DNA
   <213> Artificial
<220>
   <223> An artificially synthesized target sequence for siRNA
<400> 57
   aaacttatgg atggagcacc t 21
<210> 58
   <211> 21
   <212> DNA
   <213> Artificial
<220>
   <223> An artificially synthesized target sequence for siRNA
<400> 58
   aatcagagaa gctttactca t 21
<210> 59
   <211> 21
   <212> DNA
   <213> Artificial
<220>
   <223> An artificially synthesized target sequence for siRNA
<400> 59
   aacaaactga ctgaagataa g 21
<210> 60
   <211> 21
   <212> DNA
   <213> Artificial
<220>
   <223> An artificially synthesized target sequence for siRNA
<400> 60
   aactcgtaat gacatttcaa c 21
<210> 61
   <211> 21
   <212> DNA
   <213> Artificial
<220>
   <223> An artificially synthesized target sequence for siRNA
<400> 61
   aaaagtgatc tgcaactctt t 21
<210> 62
   <211> 21
   <212> DNA
   <213> Artificial
<220>
   <223> An artificially synthesized target sequence for siRNA
<400> 62
   aagtgatctg caactctttc a 21
<210> 63
   <211> 21
   <212> DNA
   <213> Artificial
<220>
   <223> An artificially synthesized target sequence for siRNA
<400> 63
   aactctttca ccatctgtaa t 21
<210> 64
   <211> 21
   <212> DNA
   <213> Artificial
<220>
   <223> An artificially synthesized target sequence for siRNA
<400> 64
   aactgttcga ttgtgttcaa t 21
<210> 65
   <211> 21
   <212> DNA
   <213> Artificial
<220>
   <223> An artificially synthesized target sequence for siRNA
<400> 65
   aaggatgctg atatgctaac t 21
<210> 66
   <211> 21
   <212> DNA
   <213> Artificial
<220>
   <223> An artificially synthesized target sequence for siRNA
<400> 66
   aactggtgat gagcaagtat g 21
<210> 67
   <211> 21
   <212> DNA
   <213> Artificial
<220>
   <223> An artificially synthesized target sequence for siRNA
<400> 67
   aagtatggaa ggaagttcaa g 21
<210> 68
   <211> 21
   <212> DNA
   <213> Artificial
<220>
   <223> An artificially synthesized target sequence for siRNA
<400> 68
   aacatctacc agctgaaggt g 21
<210> 69
   <211> 21
   <212> DNA
   <213> Artificial
<220>
   <223> An artificially synthesized target sequence for siRNA
<400> 69
   aagcaatgaa gaatctgaga c 21

## Description

### Technical Field

The present invention relates to the field of biological science, more specifically to the field of cancer research. In particular, the present invention relates a composition comprising a ZNFN3A1 small interfering RNA (siRNA).

### Background Art

Hepatocellular carcinoma (HCC) is among the five most frequent cancers and is the fourth leading cause of cancer death in the world. Although recent medical advances have made great progress in diagnosing the disease, a large number of patients with HCCs are still diagnosed at advanced stages. Most of the patients are not cured by surgical resection because of severe liver dysfunction, widespread and/or multiple tumors, or high incidence of recurrence. Therefore development of highly effective chemotherapeutic drugs and preventive strategies are matters of pressing concern.

### Disclosure of the Invention

The present invention based on the surprising discovery that small interfering RNAs (siRNAs) selective for ZNFN3A1 are effective for inhibiting the cellular growth of various cancer cells, including those involved in HCC.

The invention provides methods for inhibiting cell growth. Among the methods provided are those comprising contacting a cell with a composition comprising a ZNFN3A1 small interfering RNA (siRNA). The invention also provides methods for inhibiting tumor cell growth. Such methods include use of a composition comprising a ZNFN3A1 small interfering RNA (siRNA). Another aspect of the invention provides methods for inhibiting the expression of the ZNFN3A1 gene in a cell of a biological sample. Expression of the gene may be inhibited by introduction of a double stranded ribonucleic acid (RNA) molecule into the cell in an amount sufficient to inhibit expression of the ZNFN3A1 gene. Another aspect of the invention relates to products including nucleic acid sequences and vectors as well as to compositions comprising them, useful, for example, in the provided methods. Among the products provided are siRNA molecules having the property to inhibit expression of the ZNFN3A1 gene when introduced into a cell expressing said gene. Among such molecules are those that comprise a sense strand and an antisense strand, wherein the sense strand comprises a ribonucleotide sequence corresponding to a ZNFN3A1 target sequence, and wherein the antisense strand comprises a ribonucleotide sequence which is complementary to said sense strand. The sense and the antisense strands of the molecule hybridize to each other to form a double-stranded molecule.

As used herein, the term "organism" refers to any living entity comprised of at least one cell. A living organism can be as simple as, for example, a single eukaryotic cell or as complex as a mammal, including a human being.

As used herein, the term "biological sample" refers to a whole organism or a subset of its tissues, cells or component parts (e.g. body fluids, including but not limited to blood, mucus, lymphatic fluid, synovial fluid, cerebrospinal fluid, saliva, amniotic fluid, amniotic cord blood, urine, vaginal fluid and semen). "Biological sample" further refers to a homogenate, lysate, extract, cell culture or tissue culture prepared from a whole organism or a subset of its cells, tissues or component parts, or a fraction or portion thereof. Lastly, "biological sample" refers to a medium, such as a nutrient broth or gel in which an organism has been propagated, which contains cellular components, such as proteins or polynucleotides.

The invention features methods of inhibiting cell growth. Cell growth is inhibited by contacting a cell with a composition of a ZNFN3A1 small interfering RNA (siRNA). ZNFN3A1 is a zinc finger protein that is overexpressed in tumors such as hepatocellular carcinoma or colorectal adenocarcinoma. Growth of the cell expressing ZNFN3A1 can be inhibited by the present invention. The cell is further contacted with a transfection-enhancing agent. The cell is provided *in vitro, in vivo* or *ex vivo.* The subject is a mammal, *e.g*., a human, non-human primate, mouse, rat, dog, cat, horse, or cow. The cell is a hepatic cell or a colon cell. Alternatively, the cell is a tumor cell (*i.e*., cancer cell) such as a colorectal cancer cell or a liver cancer cell. For example, the cell is a colorectal adenocarcinoma cell or a hepatocellular carcinoma cell. By inhibiting cell growth is meant that the treated cell proliferates at a lower rate or has decreased viability than an untreated cell. Cell growth is measured by proliferation assays known in the art.

By the term "siRNA" is meant a double stranded RNA molecule which prevents translation of a target mRNA. Standard techniques of introducing siRNA into the cell are used, including those in which DNA is a template from which RNA is transcribed. The siRNA includes a sense ZNFN3A1 nucleic acid sequence, an anti-sense ZNFN3A1 nucleic acid sequence or both. The siRNA is constructed such that a single transcript has both the sense and complementary anti-sense sequences from the target gene, *e.g*., a hairpin.

The method is used to alter gene expression in a cell in which expression of ZNFN3A1 is upregulated, *e.g*., as a result of malignant transformation of the cells. Binding of the, siRNA to an ZNFN3A1 transcript in the target cell results in a reduction in ZNFN3A1 production by the cell. The length of the oligonucleotide is at least 10 nucleotides and may be as long as the naturally-occutring ZNFN3A1 transcript. Preferably, the oligonucleotide is 19-25 nucleotides in length. Most preferably, the oligonucleotide is less than 75, 50 , or 25 nucleotides in length. Examples of ZNFN3A1 siRNA oligonucleotides which inhibit ZNFN3A1 expression in mammalian cells include oligonucleotides containing target sequences, for example, nucleotides 451-471, 532-552, 623-643, 625-645, 636-656,726-746, 923-943,1065-1085, and 1258-1278 of SEQ ID NO:1.

Methods for designing double stranded RNA having the ability to inhibit gene expression in a target cell are known. (See for example, US Patent No. 6,506,559, . For example, a computer program for designing siRNAs is available from the Ambion website (http://www.ambion.com/techlib/misc/siRNA_finder.html).
The computer program selects nucleotide sequences for siRNA synthesis based on the following protocol.

### Selection of siRNA Target Sites

1. Beginning with the AUG start codon of the transcript, scan downstream for AA dinucleotide sequences. Record the occurrence of each AA and the 3' adjacent 19 nucleotides as potential siRNA target sites. Tuschl et al. recommend against designing siRNA to the 5' and 3' untranslated regions (UTRs) and regions near the start codon (within 75basses) as these may be richer in regulatory protein binding sites. UTR-binding proteins and/or translation initiation complexes may interfere with binding of the siRNA endonuclease complex.
2. Compare the potential target sites to the appropriate genome database (human, mouse, rat, etc.) and eliminate from consideration any target sequences with significant homology to other coding sequences. We suggest using BLAST, which can be found on the NCBI server at: www.ncbi.nlm.nih.gov/BLAST/
3. Select qualifying target sequences for synthesis. Selecting several target sequences along the length of the gene to evaluate is typical.

Also included in the invention are isolated polynucleotides that include the nucleic acid sequence of target sequences, for example, nucleotides 451-471 (SEQ NO:58), 532-552 (SEQ ID NO:60), 623-643 (SEQ ID NO:61), 625-645 (SEQ ID NO:62), 636-656 (SEQ ID NO:63), 726-746 (SEQ ID NO:64), 923-943 (SEQ ID NO:66), 1065-1085 (SEQ ID NO:68), and 1258-1278 (SEQ ID NO:69) of SEQ ID NO:1 or a polynucleotide that is complementary to the nucleic acid sequence of nucleotides 451-471, 532-552, 623-643, 625-645,636-656,726-746,923-943,1065-1085, and 1258-1278 of SEQ ID NO:1. As used herein, an "isolated nucleic acid" is a nucleic acid removed from its original environment (e.g., the natural environment if naturally occurring) and thus, synthetically altered from its natural state. In the present invention, isolated nucleic acid includes DNA, RNA, and derivatives thereof. When the isolated nucleic acid is RNA or derivatives thereof, base "t" shoulde be replaced with "u" in the nucleotide sequences. As used herein, the term "complementary" refers to Watson-Crick or Hoogsteen base pairing between nucleotides units of a polynucleotide, and the term "binding" means the physical or chemical interaction between two polypeptides or compounds or associated polypeptides or compounds or combinations thereof. Complementary nucleic acid sequences hybridize under appropriate conditions to form stable duplexes containing few or no mismatches. Furthermore, the sense strand and antisense strand of the isolated nucleotide of the present invention, can form double stranded nucleotide or hairpin loop structure by the hybridization. In a preferred embodiment, such duplexes contain no more than 1 mismatch for every 10 matches. In an especially preferred embodiment, where the strands of the duplex are fully complementary, such duplexes contain no mismatches. The polynucleotide is less than 1622 nucleotides in length. For example, the polynucleotide is less than 500, 200, or 75 nucleotides in length. Also included in the invention is a vector containing one or more of the nucleic acids described herein, and a cell containing the vectors. The isolated nucleic acids of the present invention are useful for siRNA against ZNFN3A1 or DNA encoding the siRNA. When the nucleic acids are used for siRNA or coding DNA thereof, the sense strand is preferably longer than 19 nucleotides, and more preferably longer than 21 nucleotides.

The invention is based in part on the discovery that the gene encoding a zinc finger protein, ZNFM3A1 is overexpressed in hepatocellular carcinoma (HCC) compared to non-cancerous liver tissue. The ZNFN3A1 cDNA is 1622 nucleotides in length. The 1284 ORF encodes a 428-amino acid protein with a zinc finger motif. The nucleic acid and polypeptide sequences of ZNFN3A1 are shown in Tables 1 and 2. In Table 1, the 5' and 3' untranslated region is shown in italic, the start and stop codons are in bold. The subcellular localization of ZNFN3A1 protein is altered during cell cycle progression and by the density of cultured cells. ZNFN3A1 protein accumulates in the nucleus when cells are in middle to late S phase or cultured in sparse conditions. Whereas, ZNFN3A1 protein localizes in the cytoplasm as well as in the nucleus when cells are in other phases of the cell cycle or grown in a dense condition. ZNFN3A1 forms a ternary complex with KIAA0054 protein and RNA polymerase II in *vivo,* which activates transcription of downstream genes including epidermal growth factor receptor (*EGFR*) through a direct binding of the complex with an element of "5'-CCCTCC-3"' in the 5' flanking region.

| **Table 1 Nucleic Acid Sequence of ZNFN3A1 (SEQ ID No:1)** |
|---|
| |
| |

| **Table 2 Polypeptide Sequence of ZNFN3A1 (SEQ ID NO:2)** |
|---|
| |
| |

Exogenous expression of ZNFN3A1 in NIH3T3 cells resulted in increased cell growth. In contrast, suppression of its expression with antisense S-oligonucleotides resulted in a growth-inhibition of hepatoma cells.

### Methods of inhibiting cell growth

The present invention relates to inhibiting cell growth, *i.e*, cancer cell growth by inhibiting ZNFN3A1 expression. 2NFN3A1 expression is inhibited by small interfering RNA (siRNA) that specifically target of the ZNEN3A1 gene. A ZNFN3A1 target includes, for example, nucleotides 451-471, 532-552, 623-643, 625-645, 636-656, 726-746, 923-943, 1065-1085, and 1258-1278 of SEQ ID NO:1.

In non-mammalian cells, double-stranded RNA (dsRNA) has been shown to exert a strong and specific silencing effect on gene expression, which is referred as RNA interference (RNAi) (3). dsRNA is processed into 20-23 nucleotides dsRNA called small interfering RNA (siRNA) by an enzyme containing RNase III motif. The siRNA specifically targets complementary mRNA with a multicomponent nuclease complex (4, 5). In mammalian cells, siRNA composed of 20 or 21-mer dsRNA with 19 complementary nucleotides and 3' terminal noncomplementary dimmers of thymidine or uridine, have been shown to have a gene specific knock-down effect without inducing global changes in gene expression (6). In addition, plasmids containing small nuclear RNA (snRNA) U6 or polymerase III H1-RNA promoter effectively produce such short RNA recruiting type III class of RNA polymerase III and thus can constitutively suppress its target mRNA (7, 8).

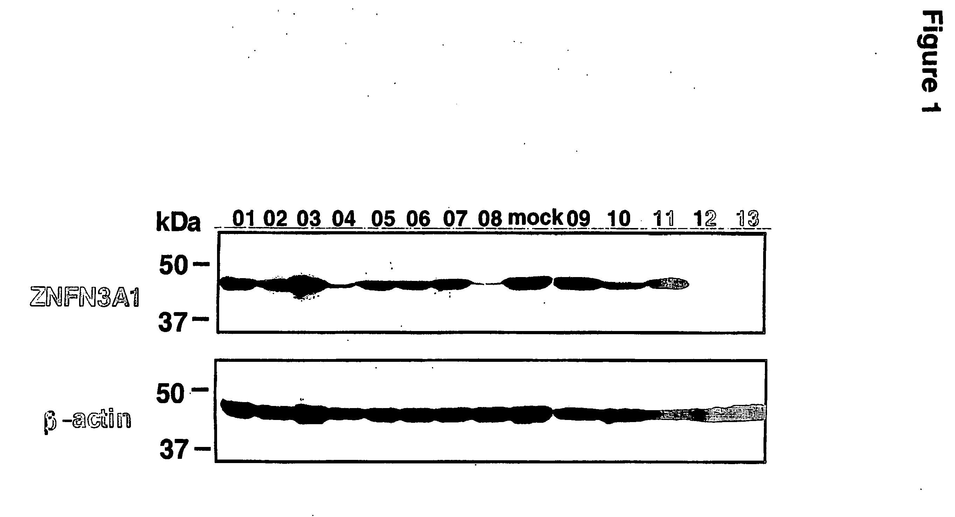

13 different expression plasmids were constructed to express hairpin-looped ZNFN3A1-siRNA (See Example 2). The plasmids were tested for their ability to inhibit cell growth. Four plasmids (psiU6BX-ZNFN3A1-4, -8, -12 and -13) markedly and five plasmids (psiU6BX-ZNFN3A1-2, -5, -6, -7, and -10) moderately suppressed endogeneous ZNFN3A1 expression, while the remaining four plasmids (psiU6BX-ZNFN3A1-1, -3, -9 and -11 exhibited no or little effect on the expression. (Figure 1). Various human hepatoma and colorectal cancer cells transfected with psiU6BX-siZNFN3A1-12, showed reduced number of surviving cells compared to control plasmids. FACS analysis revealed that their death was due to apoptosis.

The growth of cells are inhibited by contacting a cell, with a composition containing a ZNFN3A1 siRNA. The cell is further contacted with a transfection agent. Suitable transfection agents are known in the art. By inhibition of cell growth is meant the cell proliferates at a lower rate or has decreased viability compared to a cell not exposed to the composition. Cell growth is measured by methods known in the art such as, the MTT cell proliferation assay.

The ZNFN3A1-siRNA is directed to a single target ZNFN3A1 gene sequence. Alternatively, the siRNA is directed to multiple target ZNFN3A1 gene sequences. For example, the composition contains ZNFN3A1- siRNA directed to two, three, four, or five or more ZNFN3A1 target sequences. By ZNFN3A1 target sequence is meant a nucleotide sequence that is identical to a portion of the ZNFN3A1 gene. The target sequence can include the 5' untranslated (UT) region, the open reading frame (ORF) or the 3' untranslated region of the human ZNFN3A1 gene. Alternatively, the siRNA is a nucleic acid sequence complementary to an upstream or downstream modulator of ZNFN3A1 gene expression. Examples of upstream and downstream modulators include, a transcription factor that binds the ZNFN3A1 gene promoter, a kinase or phosphatase that interacts with the ZNFN3A1 polypeptide, a ZNFN3A1 promoter or enhancer.

ZNFN3A1- siRNA which hybridize to target mRNA decrease or inhibit production of the ZNFN3A1 polypeptide product encoded by the ZNFN3A1 gene by associating with the normally single-stranded mRNA transcript, thereby interfering with translation and thus, expression of the protein. The siRNA is less than 500, 200, 100, 50, or 25 nucleotides in length. Preferably the siRNA is 19-25 nucleotides in length. Exemplary nucleic acid sequence for the production of ZNFN3A1-siRNA include the sequences of nucleotides 451-471 (SEQ ID NO:58), 532-552 (SEQ ID NO:60), 623-643 (SEQ ID NO:61), 625-645 (SEQ ID NO:62), 636-656 (SEQ ID NO:63), 726-746 (SEQ ID NO:64), 923-943 (SEQ ID NO:66), 1065-1085 (SEQ ID NO:68), or 1258-1278 (SEQ ID NO:69) of SEQ ID NO:1 as the target sequence. Furthermore, in order to enhance the inhibition activity of the siRNA, nucleotide "u" can be added to 3'end of the antisense strand of the target sequence. The number of "u"s to be added is at least 2, generally 2 to 10, preferably 2 to 5. The added "u"s form single strand at the 3'end of the antisense strand of the siRNA.

The cell is any cell that expresses or over-expresses ZNFN3A1. The cell is a hepatic cell or an epithelial cell such as a colon cell. Alternatively, the cell is a tumor cell such as a carcinoma, adenocarcinoma, blastoma, leukemia, myeloma, or sarcoma. The cell is a hepatocellular carcinoma or a colorectal adenocarcinoma cell.

An ZNFN3A1-siRNA is directly introduced into the cells in a form that is capable of binding to the mRNA transcripts. Alternatively, the DNA encoding the ZNFN3A1-siRNA is in a vector.

Vector are produced for example by cloning a ZNFN3A1 target sequence into an expression vector operatively-linked regulatory sequences flanking the ZNFN3A1 sequence in a manner that allows for expression (by transcription of the DNA molecule) of both strands (Lee, N.S., Dohjima, T., Bauer, G., Li, H., Li, M.-J., Ehsani, A.,Salvaterra, P., and Rossi, J. (2002) Expression of small interfering RNAs targeted against HIV-1 rev transcripts in human cells. Nature Biotechnology 20 : 500-505.). An RNA molecule that is antisense to ZNFN3A1 mRNA is transcribed by a first promoter (*e.g*., a promoter sequence 3' of the cloned DNA) and an RNA molecule that is the sense strand for the ZNFN3A1 mRNA is transcribed by a second promoter (*e.g*., a promoter sequence 5' of the cloned DNA). The sense and antisense strands hybridize *in vivo* to generate siRNA constructs for silencing of the ZNFN3A1 gene. Alternatively, two constructs are utilized to create the sense and anti-sense strands of a siRNA construct. Cloned ZNFN3A1 can encode a construct having secondary structure, e.g., hairpins, wherein a single transcript has both the sense and complementary antisense sequences from the target gene.
A loop sequence consisting of an arbitrary nucleotide sequence can be located between the sense and antisense sequence in order to form the hairpin loop structure. Thus, the present invention also provides siRNA having the general formula 5'-[A]-[B]-[A']-3', wherein [A] is a ribonucleotide sequence corresponding to a sequence selected from the group consisting of nucleotides 451-471 (SEQ ID NO:58). 532-552 (SEQ ID NO:60), 623-643 (SEQ ID NO:61), 625-645 (SEQ ID NO:62), 636-656 (SEQ ID NO:63), 726-746 (SEQ ID NO:64), 923-943 (SEQ ID NO:66), 1065-1085 (SEQ ID NO:68), and 1258-1278 (SEQ ID NO:69) of SEQ ID NO:1,
[B] is a ribonucleotide sequence consisting of 3 to 23 nucleotides, and
[A'] is a ribonucleotide sequence consisting of the complementary sequence of [A]

The region [A] hybridizes to [A'], and then a loop consisting of region [B] is formed. The loop sequence may be preferably 3 to 23 nucleotide in length. The loop sequence, for example, can be selected from group consisting of following sequences (http://www.ambion.com/techlib/tb/tb_506.html). Furthermore, loop sequence consisting of 23 nucleotides also provides active siRNA (Jacque, J.-M., Triques, K., and Stevenson, M. (2002) Modulation of HIV-1 replication by RNA interference. Nature 418 : 435-438.).

AUG :Sui, G., Soohoo, C., Affar, E.B., Gay, F., Shi, Y., Forrester, W.C., and Shi, Y. (2002) A DNA vector-based RNAi technology to suppress gene expression in mammalian cells. Proc. Natl. Acad. Sci. US A 99(8): 5515-5520.

CCC, CCACC or CCACACC: Paul, C.P., Good, P.D., Winer, I., and Engelke, D.R. (2002) Effective expression of small interfering RNA in human cells. Nature Biotechnology 20 : 505-508.

UUCG: Lee, N.S., Dohjima, T., Bauer, G., Li, H., Li, M.-J., Ehsani, A., Salvaterra, P., and Rossi, J. (2002) Expression of small interfering RNAs targeted against HIV-1 rev transcripts in human cells. Nature Biotechnology 20 : 500-505.

CTCGAG or AAGCUU: Editors of Nature Cell Biology (2003) Whither RNAi? Nat Cell Biol. 5:489-490.

UUCAAGAGA: Yu, J.-Y., DeRuiter, S.L., and Turner, D.L. (2002) RNA interference by expression of short-interfering RNAs and hairpin RNAs in mammalian cells. Pro. Natl. Acad. Sci. USA 99(9): 6047-6052.

For example, preferable siRNAs having hairpin loop structure of the present invention are shown below. In the following structure, the loop sequences can be selected from group consisting of AUG, CCC, UUCG, CCACC, CTCGAG, AAGCUU, CCACACC, and UUCAAGAGA. Preferable loop sequence is UUCAAGAGA ("ttcaagaga" in DNA).
aaucagagaagcuuuacucau-[B]-augaguaaagcuucucugauu (for target sequence of SEQ ID NO:58)
aacucguaaugacauuucaac-[B]-guugaaaugucauuacgaguu (for target sequence of SEQ ID NO:60)
aaaagugaucugcaacucuuu-[B]-aaagaguugcagaucacuuuu (for target sequence of SEQ ID NO:61)
aagugaucugcaacucuuuca-[B]-ugaaagaguugcagaucacuu (for target sequence of SEQ ID NO:62)
aacucuuucaccaucuguaau-[B]-auuacagauggugaaagaguu (for target sequence of SEQ ID NO:63)
aacuguucgauuguguucaau-[B]-auugaacacaaucgaacaguu (for target sequence of SEQ ID NO:64)
aacuggugaugagcaaguaug-[B]-cauacuugcucaucaccaguu (for target sequence of SEQ ID NO:66)
aacaucuaccagcugaaggug-[B]-caccuucagcugguagauguu (for target sequence of SEQ ID NO:68)
aagcaaugaagaaucugagac-[B]-gucucagauucuucauugcuu (for target sequence of SEQ ID NO:69)

The regulatory sequences flanking the ZNFN3A1 sequence are identical or are different, such that their expression can be modulated independently, or in a temporal or spatial manner. siRNAs are transcribed intracellularly by cloning the ZNFN3A1 gene templates into a vector containing, *e.g*., a RNA pol III transcription unit from the small nuclear RNA (snRNA) U6 or the human H1 RNA promoter. For introducing the vector into the cell, transfection-enhancing agent can be used. FuGENE (Rochediagnostices), Lipofectamin 2000 (Invitrogen), Oligofectamin (Invitrogen), and Nucleofactor (Wako pure Chemical) are useful as the transfection-enhancing agent.

Oligonucleotides and oligonucleotides complementary to various portions of ZNFN3A1 mRNA were tested *in vitro* for their ability to decrease production of ZNFN3A1 in tumor cells (e.g., using the Alexander and HepG2 hepatocellular carcinoma (HCC) cell line and the HCT116 and SW948 colorectal cancer cell line) according to standard methods. A reduction in ZNFN3A1 gene product in cells contacted with the candidate siRNA composition compared to cells cultured in the absence of the candidate composition is detected using ZNFN3A1-specific antibodies or other detection strategies. Sequences which decrease production of ZNFN3A1 in *in vitro* cell-based or cell-free assays are then tested for there inhibitory effects on cell growth. Sequences which inhibit cell growth in *in vitro* cell-based assay are test in in *vivo* in rats or mice to confirm decreased ZNFN3A1 production and decreased tumor cell growth in animals with malignant neoplasms.

### Methods of treating malignant tumors

Patients with tumors characterized as over-expressing ZNFN3A1 are treated by administering ZNFN3A1-siRNA. siRNA therapy is used to inhibit expression of ZNFN3A1 in patients suffering from or at risk of developing, for example, hepatocellular carcinomas, or colorectal cancer. Such patients are identified by standard methods of the particular tumor type. Hepatocellular carcinoma is diagnosed for example, by enlargement of the liver, tomography, ultrasound or biopsy. Colorectal cancer is diagnosed for example, by blood in stool, colonoscopy, flexible sigmoidoscopy, CEA Assay, double contrast barium enema CT Scan, tomography or biopsy.

Treatment is efficacious if the treatment leads to clinical benefit such as, a reduction in expression of ZNFN3A1, or a decrease in size, prevalence, or metastatic potential of the tumor in the subject. When treatment is applied prophylactically, "efficacious" means that the treatment retards or prevents tumors from forming or prevents or alleviates a symptom of clinical symptom of the tumor. Efficaciousness is determined in association with any known method for diagnosing or treating the particular tumor type.

siRNA therapy is carried out by administering to a patient a siRNA by standard vectors and/or gene delivery systems. Suitable gene delivery systems may include liposomes, receptor-mediated delivery systems, or viral vectors such as herpes viruses, retroviruses, adenoviruses and adeno-associated viruses, among others. A reduction in ZNFN3A1 production results in a decrease ZNFN3A1 complex formation with KIAA0054 protein and RNA polymerase II or a decrease in ZNFN3A1 protein expression. A therapeutic nucleic acid composition is formulated in a pharmaceutically acceptable carrier. The therapeutic composition may also include a gene delivery system as described above. Pharmaceutically acceptable carriers are biologically compatible vehicles which are suitable for administration to an animal, *e.g*., physiological saline. A therapeutically effective amount of a compound is an amount which is capable of producing a medically desirable result such as reduced production of a ZNFN3A1 gene product, reduction of cell growth, *e.g*., proliferation, or a reduction in tumor growth in a treated animal.

Parenteral administration, such as intravenous, subcutaneous, intramuscular, and intraperitoneal delivery routes, may be used to deliver ZNFN3A1-siRNA compositions. For treatment of hepatic tumors, direct infusion the portal vein is useful.

Dosages for any one patient depends upon many factors, including the patient's size, body surface area, age, the particular nucleic acid to be administered, sex, time and route of administration, general health, and other drugs being administered concurrently. Dosage for intravenous administration of nucleic acids is from approximately 10⁶ to 10²² copies of the polynucleotide.

The polynucleotides are administered by standard methods, such as by injection into the interstitial space of tissues such as muscles or skin, introduction into the circulation or into body cavities or by inhalation or insufflation. Polynucleotides are injected or otherwise delivered to the animal with a pharmaceutically acceptable liquid carrier, *e.g*., a liquid carrier, which is aqueous or partly aqueous. The polynucleotides are associated with a liposome (*e.g*., a cationic or anionic liposome). The polynucleotide includes genetic information necessary for expression by a target cell, such as a promoters.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting

### Brief Description of the Drawings

Fig. 1 is a photograph of an immunoblot showing the effect of ZNFN3A1 siRNAs on exogeneous ZNFN3A1 expression in COS7 cells.
Fig. 2 is a photograph of an immunoblot showing the expression of ZNFN3A1 protein in hepatoma and colon cancer cell lines.
Fig. 3 is a photograph of an immunoblot showing the effect of ZNFN3A1-siRNAs on endogeneous ZNFN3A1 expression in SNU475 cell transfected with psiU6BX-ZNFN3A1-1, -4, -12 or psiU6BX-mock plasmids.
Fig. 4A-B are bar charts showing the effect of ZNFN3A1-siRNAs on cell growth in SNU475 cells. Viability of transfected cells was measured by MTT assay 6 (Panel A) and 9 (panel B) days after the transfection.
Fig. 5 are bar charts showing growth suppressive effect of ZNFN3A1-siRNAs in various human hepatoma and colon cancer cells. Viability of transfected cells was measured by MTT assay, 9 to 12 days after the transfection.
Figure 6 is an illustration showing cell death in response to ZNFN3A1-siRNAs in SNU475 cell detected by FACS analysis.

### Best Mode for Carrying out the Invention

The invention will be further described in the following examples, which do not limit the scope of the invention described in the claims.

### [Example 1] General Methods

### Cell lines and tissue specimens

Human hepatoma cell lines Alexander and HepG2, human colon cancer lines HCT116 and SW948, and monkey fibroblast cell line COS7 were obtained from the American Type Culture Collection (ATCC). Human hepatoma cell line Huh7 was obtained from Japanese Collection of Research Bioresources (JCRB). Human hepatoma cell lines, SNU398, SNU423, SNU449 and SNU475 were obtained from the Korea cell-line bank. All these cells are publicly available.

All cell lines were grown in monolayers in appropriate media: Dulbecco's modified Eagle's medium for Alexander, Huh7, HepG2 and COS7; McCoy's 5A for HCT116; Leibovitz's L-15 for SW948; RPMI1640 for SNU398, SNU423, SNU449 and SNU475 supplemented with 10% fetal bovine serum and 1% antibiotic/antimycotic solution (Sigma). All cells were maintained at 37°C in humid air with 5% CO₂. (Alexander, Huh7, HepG2, SNU348, SNU423, SNU449, SNU475, HCT116, and COS7) or without CO₂(SW948).

### Cloning of ZNFN3A1

Cloning of *ZNFN3A1* was done by PCR using KOD-plus (TOYOBO). For E. Coli expression, coding region of *ZNFN3A1* was cloned in the *Eco*R I*-Kpn* I site of pET21a. For mammalian cell expression, coding region of *ZNFN3A1* was cloned in the *Eco*R I-K*pn* I site of pcDNA3.1 (+) and (-) (Invitrogen), *Eco*R I-*Kpn* I site ot pFLAG and *Eco*R I-*Kpn* I site of pEGFP (Clontech). Coding region of *KIAA0054* was cloned in the *Eco*R I-*Xho* I site of pCMV-HA (Clontech).

### ZNFN3A1 Polyclonal Antibody Production

Rabbit anti-ZNFN3A1 polyclonal antibody was generated. Full coding sequence of *ZNFN3A1* was amplified by PCR reaction using testis cDNA as a template and cloned in pET21 a (Novagen). The cloned vector was transfected into BL21-CodonPlus® competent cells (Stratagene). Recombinant ZNFN3A1 protein was induced by 1.0 mM IPTG at 30°C for 6 h. His-ZNFN3A1 fusion protein was purified using Pro Bond™ Resin (Invitrogen). Rabbits were immunized ten times with purified His-ZNFN3A1. Immunoblotting with this polyclonal antibody showed single 50 kD band of FLAG-tagged ZNFN3A1, which was identical pattern to that detected using anti-FLAG monoclonal antibody (Sigma) (data not shown).

### RNA preparation and RT-PCR

Total RNA was extracted with Trizol reagent (Life technologies) according to the manufacturer's protocol. Ten-microgram aliquots of total RNA were reversely transcribed for single-stranded cDNAs using poly dT₁₂₋₁₈ primer (Amersham Biosciences) with Superscript II reverse transcriptase (Life Technologies). Each single-stranded cDNA was diluted for subsequent PCR amplification. Standard RT-PCR was carried out in a 20 µl volume of PCR buffer (TAKARA), and amplified for 4 min at 94°C for denaturing, followed by 20 (for *GAPDH*) or 30 (for *ZNFN3A1*) cycles of 94°C for 30 s, 56°C for 30 s, 72°C for 30 s, in the Gene Amp PCR system 9700 (Perkin-Elmer). Primer sequences were as follows,
for *GAPDH*
forward; 5'-ACAACAGCCTCAAGATCATCAG-3' (SEQ ID No: 29) and
reverse; 5'-GGTCCACCACTGACACGTTG-3' (SEQ ID No: 30),
for or *ZNFN3A1*
forward; 5'-TTCCCGATATCAACATCTACCAG-3' (SEQ ID No: 31) and
reverse; 5'-AGTGTGTGACCTCAATAAGGCAT-3' (SEQ ID No: 32).

### Construction of psiU6BX6 Plasmid

The DNA flagment encoding siRNA was inserted into the GAP at nucleotide 485-490 as indicated (-) in the following plasmid sequence (SEQ ID No: 33).

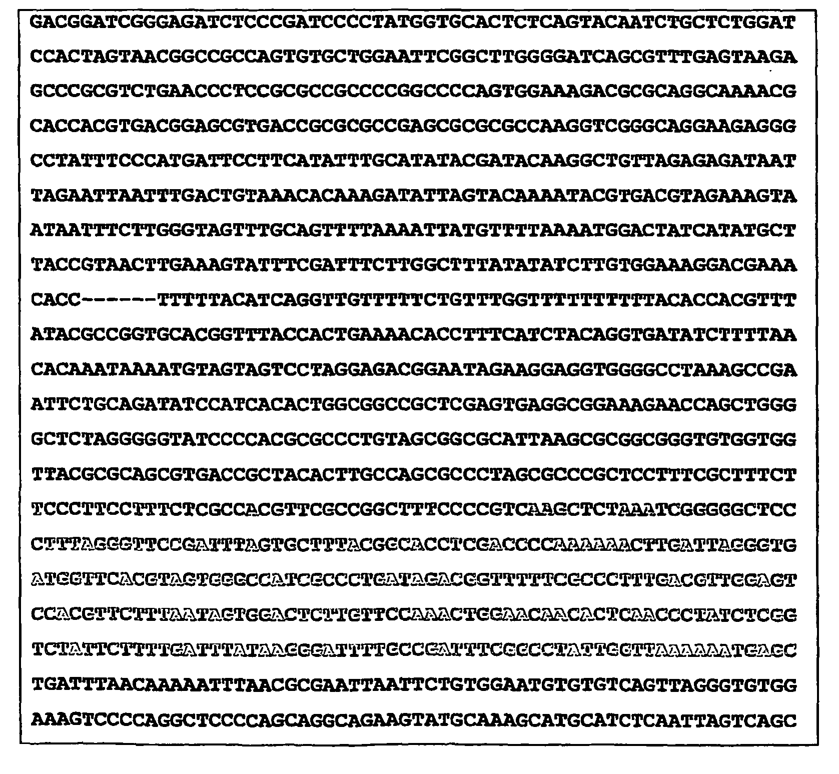

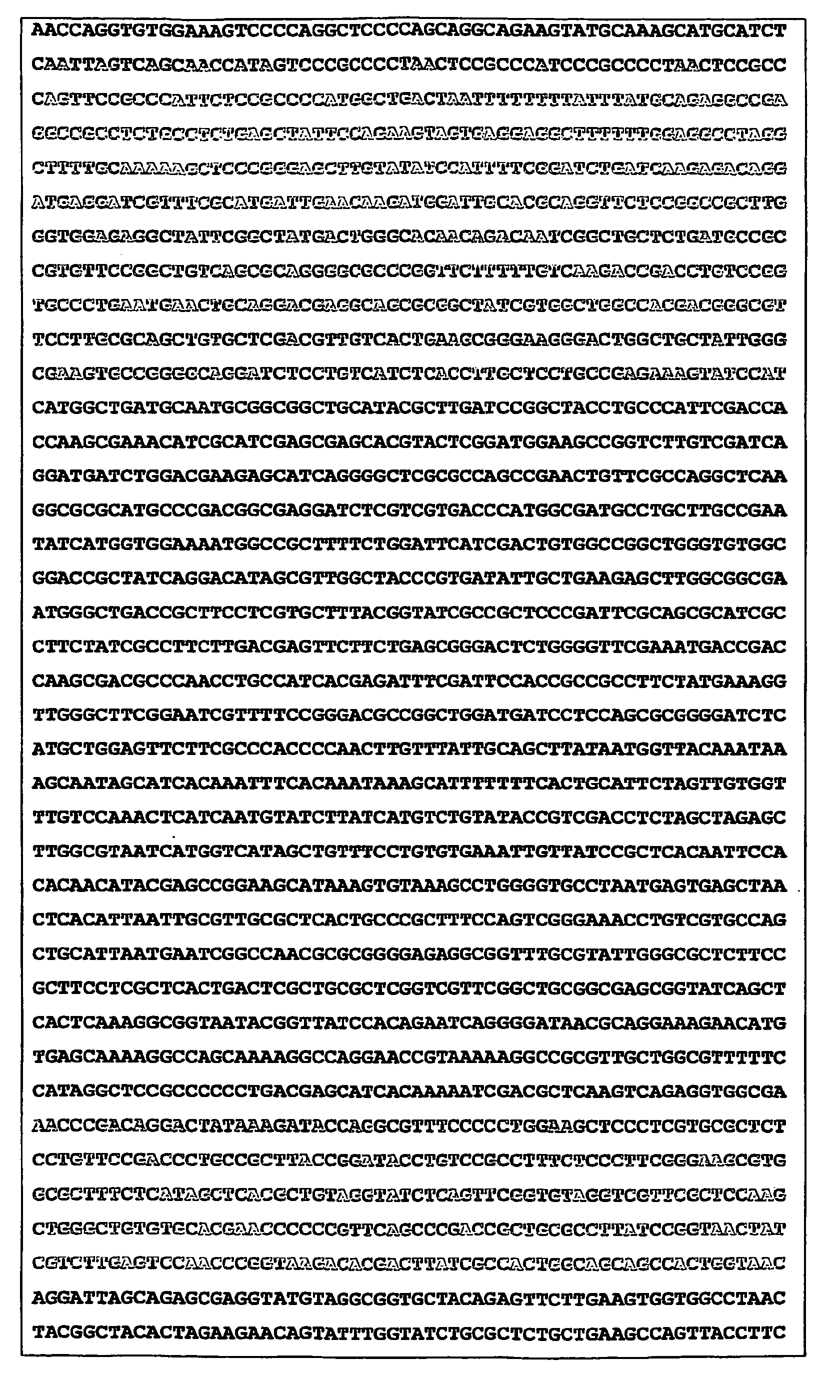

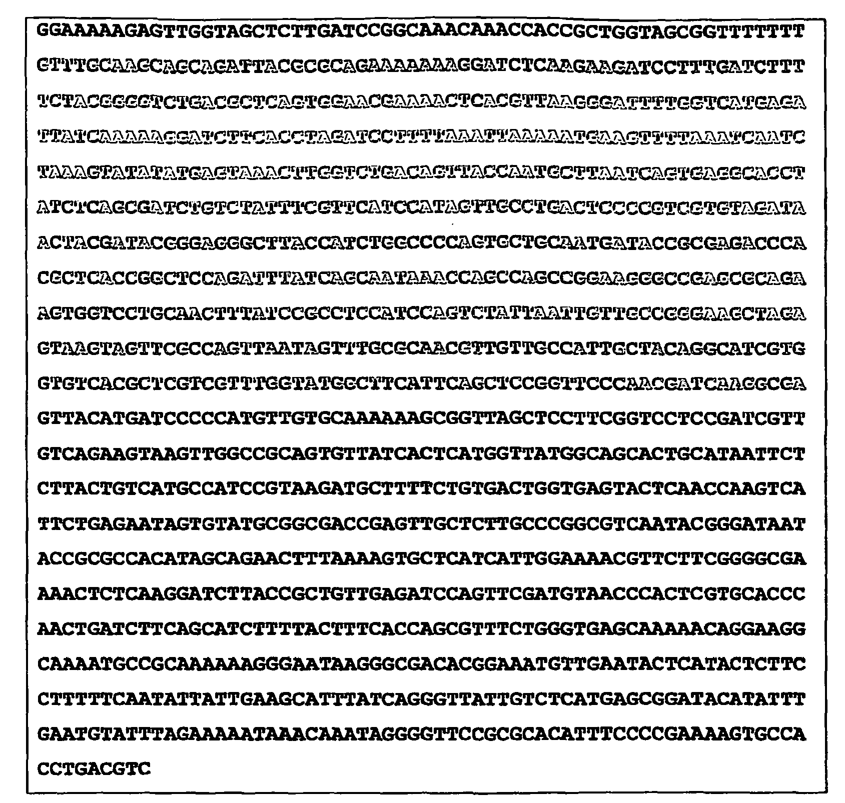

snRNA U6 gene is reported to be transcribed by RNA polymerase III, which produce short transcripts with uridines at the 3' end. The genomic fragment of the snRNA U6 gene containing the promoter region was amplified by PCR using a set of primers,
5'-GGGGATCAGCGTTTGAGTAA-3' (SEQ ID No: 34), and
5'-TAGGCCCCACCTCCTTCTAT-3' (SEQ ID No: 35) and human placental DNA as a template. The product was purified and cloned into pCR plasmid vector using a TA cloning kit according to the supplier's protocol (Invitrogen). The *Bam*HI, *Xho*I fragment containing the siRNA U6 gene was purified and cloned into nucleotide 1257 to 56 fragment of pcDNA3.1(+) plasmid, which was amplified by PCR with a set of primer, 5'-TGCGGATCCAGAGCAGATTGTACTGAGAGT-3' (SEQ ID No: 36) and 5'-CTCTATCTCGAGTGAGGCGGAAAGAACCA-3' (SEQ No: 37). The ligated DNA was used for a template of PCR with primers,
5'-TTTAAGCTTGAAGACTATTTTTACATCAGGTTGTTTTTCT-3' (SEQ ID No: 38) and
5'-TTTAAGCTTGAAGACACGGTGTTTCGTCCTTTCCACA-3' (SEQ ID No: 39). The product was digested with HindIII, which was subsequently self-ligated to produce psiU6BX vector plasmid. For the control, psiU6BX-EGFP was prepared by cloning double-stranded oligonucleotides of
5'- CACCGAAGCAGCACGACTTCTTCTTCAAGAGAGAAGAAGTCGTGCT GCTTC-3' (SEQ ID No: 40) and
5'-AAAAGAAGCAGCACGACTTCTTCTCTCTTGAAGAAGAAGTCGTGCT GCTTC -3' (SEQ ID No: 41) into the Bbsl site in the psiU6BX vector.

### Immunoblotting

The polyclonal antibody to ZNFN3A1 was previously purified from sera of immunized rabbits with recombinant His-tagged ZNFN3A1 protein. Proteins were separated by 10% SDS-PAGE and immunoblotted with the anti-ZNFN3A1 antibody. HRP-conjugated goat anti-rabbit IgG (Santa Cruz Biotechnology, Santa Cruz, CA) served as the secondary antibody for the ECL Detection System (Amersham Pharmacia Biotech, Piscataway, NJ). Immunoblotting with the anti-ZNFN3A1 antibody showed single 50 kD band of FLAG-tagged ZNFN3A1, which was identical pattern to that detected using anti-FLAG antibody

### 3-(4,5-dimethylthiazol-2-yl)-2,5-diphenyltetrazolium bromide (MTT) assay

Cells were transfected with psiU6BX-siZNFN3A1 or control plamids and maintained in the culture media supplemented with optimum concentration of geneticin. Six to twelve days after transfection, the medium was replaced with fresh medium containing 500 µg/ml of MTT (3-(4,5-dimethylthiazol-2-yl)-2,5-diphenyl tetrazolium bromide) (Sigma) and the plates were incubated for four hours at 37°C. Subsequently, the cells were lysed by the addition of 1 ml of 0.01 N HCl/10%SDS and. absorbance of lysates was measured with an ELISA plate reader at a test wavelength of 570 nm (reference, 630 nm). The cell viability was represented by the absorbance compared to that of control cells.

### Flow cytometry

The effect of ZNFN3A1 in cell cycle progression was determined by flow cytometry. Cells were plated at a density of 1X10⁵ cells/100 mm dish. The cells were trypsinized at the given time course, collected in PBS and fixed in 70% cold ethanol. After RNase treatment, cells were stained with propidium iodide (50 µg/ml) in PBS. Flow cytometry was performed on a Becton Dickinson FACScan and analyzed by CellQuest and ModFit software (Verity Software House), The percentages of nuclei in G0/G1, S and G2/M phases of the cell cycle, and any sub-G1 population were determined from at least 20,000 ungated cells.

To examine the role of ZNFN3A1-siRNAs in cell cycle, 1X10⁵ of SNU475 cells transfected with psiU6BX-ZNFN3A1 or control plasmids were collected by trypsinization at 5 days after transfection. After fixation in 70% cold ethanol, cells were treated with RNase and propidium iodide (50 µg/ml) in PBS, and analyzed by a FACScan (Becton Dickinson, San Jose, CA). The percentages of cells in G0/G1, S and G2/M phases of the cell cycle, and any sub-G1 population were determined from at least 20,000 ungated cells using ModFit software (Verity Software House)

### [Example 2] Production and Characterization of Plasmids Expressing ZNFN3A1 siRNAs

The entire coding sequence of *ZNFN3A1* was amplified with a set of primers, 5'-GGGGTACCAGGATGGAGCCGCTGAAGGTGG-3' (SEQ ID No: 42), and 5'-GGGAATTCTTAGGATGCTCTGATGTTGGCGTCG-3' (SEQ ID No: 43) and cloned into the appropriate cloning sites of pcDNA 3.1(+) vector (Invitrogen) (pcDNA-ZNFN3A1). Plasmids expressing ZNFN3A1-siRNAs were prepared by cloning of double-stranded oligonucleotides into psiU6BX vector.

The nucleotide sequence of the siRNAs were designed using an siRNA design computer program available from the Ambion website.
(http://www.ambion.com/techlib/misc/siRNA_finder.html). Briefly, nucleotide sequences for siRNA synthesis are selected using the following protocol.

### Selection of siRNA Target Sites:

1. Starting with the AUG start codon of the ZNFN3A1 transcript, scan downstream for an AA dinucleotide sequences. The occurrence of each AA and the 3' adjacent 19 nucleotides are recorded as potential siRNA target sites. Tuschl et al. recommend against designing siRNA to the 5' and 3' untranslated regions (UTRs) and regions near the start codon (within 75bases) as these may be richer in regulatory protein binding sites. UTR-binding proteins and/or translation initiation complexes may interfere with binding of the siRNA endonuclease complex.
2. The potential target sites are compared to the appropriate genome database (human, mouse, rat, etc.) to eliminate target sequences with significant homology to other coding sequences.
3. Qualifying target sequences are selected for synthesis. Several target sequences along the length of the gene are selected for evaluation.
The oligonucleotides used for ZNFN3A1 siRNAs are shown below. psiU6BX-ZNFN3A1 1-13 (siRNA 1-13) were prepared by cloning the following double-stranded oligonucleotide into the Bbsl site of the psiU6 vector. The corresponding nucleotide position relative to the ZNFN3A1 nucleic acid sequence of SEQ ID NO:1 is listed for each oligonucleotide sequence. Each oligionucleotide is a combination of a sense nucleotide sequence and an antisense nucleotide sequence of the target sequence ZNFN3A1. The nucleotide sequences of the hairpin loop structure and target sequence of siRNA1 to 13 are shown in SEQ ID NO:44 to SEQ ID NO:56 and SEQ ID NO:57 to SEQ ID NO:69, respectively (endonuclease recognition cites are eliminated from each hairpin loop structure sequence).

psiU6BX-ZNFN3A1-1 /siRNA1: (nucleotide numbers 426-446 of SEQ ID No: 1) and

psiU6BX-ZNFN3A1-2 /siRNA2: (nucleotide numbers 451-471 of SEQ ID No: 1) and

psiU6BX-ZNFN3A1-3/siRNA3: (nucleotide numbers 495-515 of SEQ ID No: 1) and

psiU6BX-ZNFN3A1-4 /siRNA4: (nucleotide numbers 532-552 of SEQ ID No: 1) and

psiU6BX-ZNFN3A1-5 /siRNA5: (nucleotide numbers 623-643 of SEQ ID No: 1) and

psiU6BX-ZNFN3A1-6 /siRNA6: (nucleotide numbers 625-645 of SEQ ID No: 1) and

psiU6BX-ZNFN3A1-7 /siRNA7: (nucleotide numbers 636-656 of SEQ ID No: 1) and

psiU6BX-ZNFN3A1-8 /siRNA8: (nucleotide numbers 726-746 of SEQ ID No: 1) and

psiU6BX-ZNFNA1-9 /siRNA9: (nucleotides numbers 906-926 of SEQ ID No: 1) and

psiU6BX-ZNFN3A1-10 /siRNA10: (nucleotide numbers 923-943 of SEQ ID No: 1) and

psiU6BX-ZNFN3A1-11 /siRNA11: (nucleotide numbers 937-957 of SEQ ID No: 1) and

psiU6BX-ZNFN3A1-12 /siRNA12: (nucleotide numbers 1065-1085 of SEQ ID No: 1) and

psiU6BX-ZNFN3A1-13 /siRNA13: (nucleotide numbers 1258-1278 of SEQ ID No: 1) and

psiU6BX-siZNFN3A1 or psiU6BX-mock plasmids were transfected with pcDNA-ZNFN3A1 into COS7 cells using FuGENE6 reagent according to the supplier's recommendations (Roche). The plasmids where solely transfected into SNU479 cells expressing abundant amount of endogeneous ZNFN3A1. Whole extracts of the cells were lysed 2 days after the transfection and utilized for immunoblot analysis.

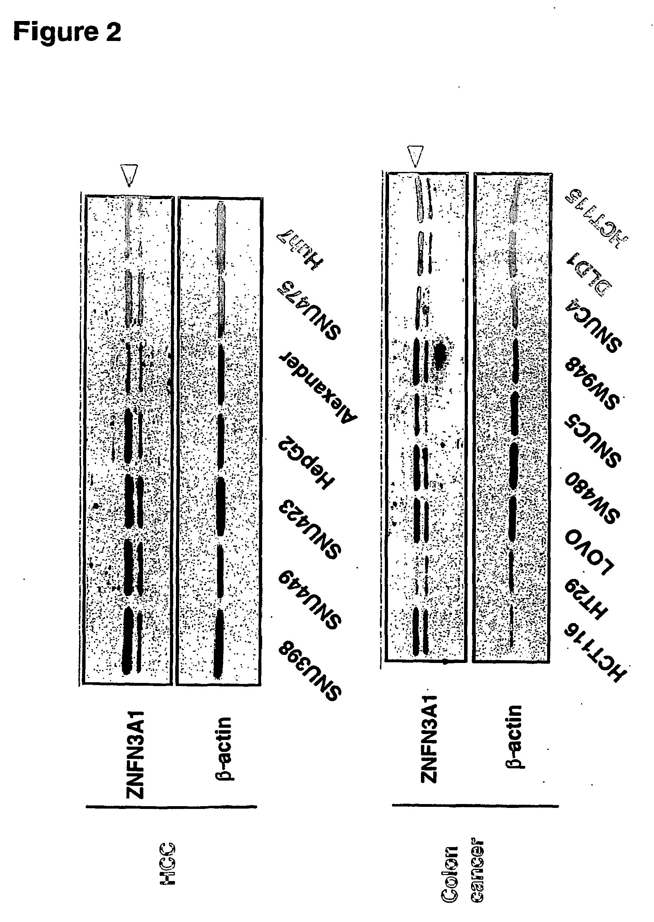

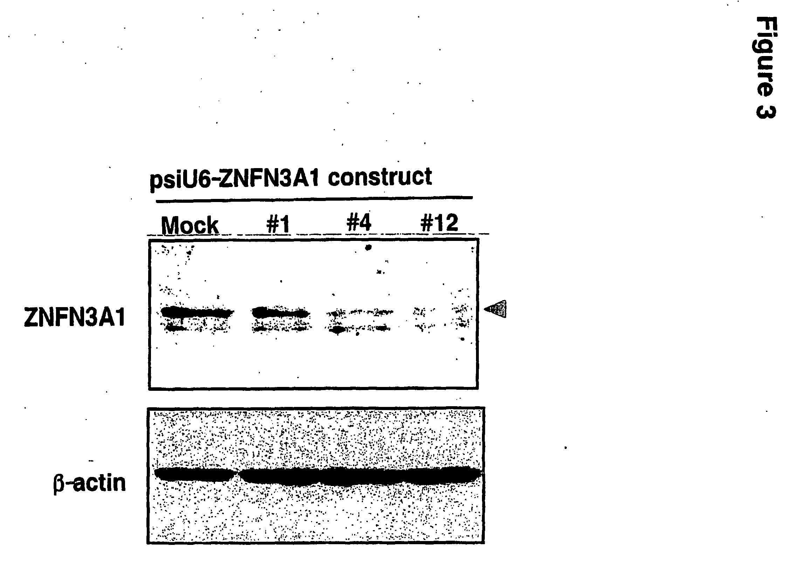

Among the 13 different expression plasmids expressing ZNFN3A1 siRNAs, psiUGBX-ZNFN3A1-8, -12, and -13 most significantly reduced expression of exogeneous ZNFN3A1 by western blot analysis, when they were transfected into COS7 cells together with pcDNA-ZNFN3A1. Among other plasmids, psiUGBX-ZNFN3A1-4 showed marked reduction, and psiU6BX-ZNFN3A1-2, -5, -6, -7 and -10 exerted moderate suppression, whereas psiU6BX-ZNFN3A1-1, -3, -9 and -11 had no or little effect on the expression (Figure 1). To further examine RNAi activity of ZNFN3A1 siRNAs, we transfected psiU6BX-ZNFN3A1-1, -4, -12, or psiU6BX-mock into SNU475 cells that express abundant amount of ZNFN3A1 (Figure 2). Western blot analysis using the extracts of transfected cells demonstrated marked reduction of endogeneous ZNFN3A1 by psiU6BX-ZNFN3A1-12, and moderate suppression by psiU6BX-ZNFN3A1-4 compared to cells transfected with psiU6BX-mock. On the other hand transfection with psiU6BX-ZNFN3A1-1 did not affect expression of ZNFN3A1 (Figure 3).

### [Example 3] Growth suppression of hepatoma and colon cancer cells by ZNFN3A1 siRNA

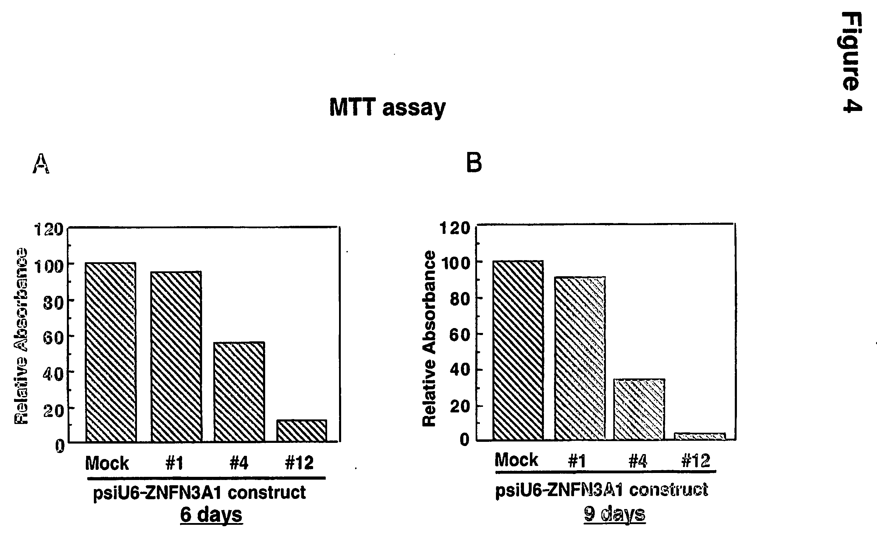

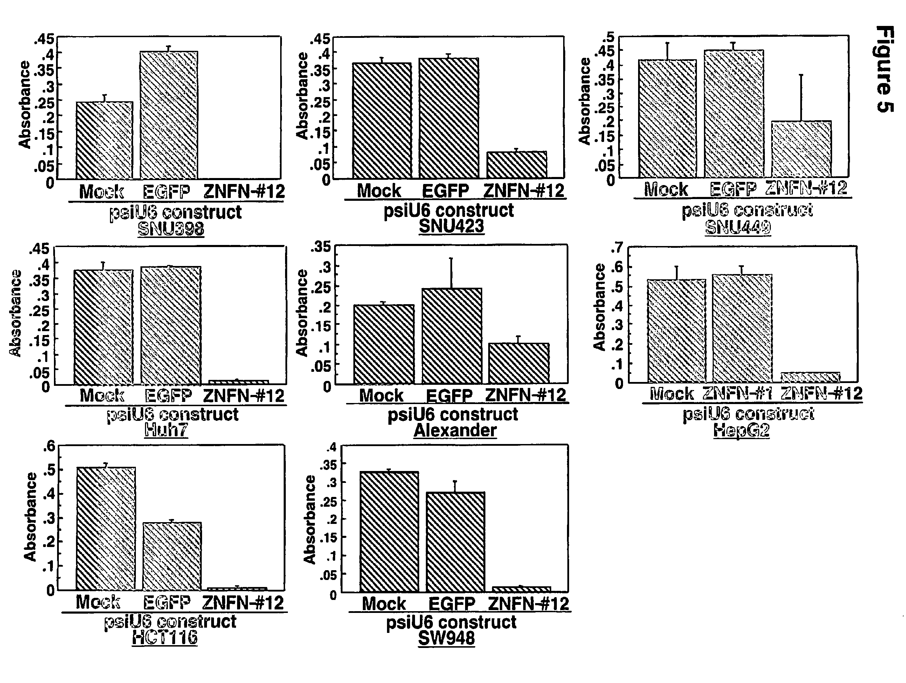

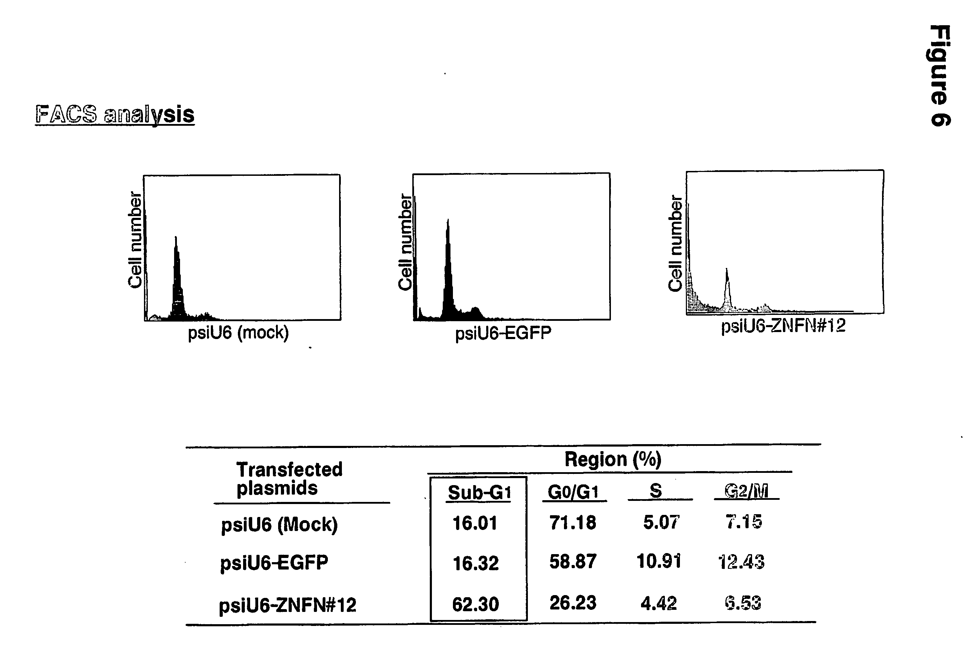

To test whether suppression of *ZNFN3A1* may result in growth suppression of hepatoma cells, SNU475 cells were transfected with either psiU6BX-ZNFN3A1-12, the vector that demonstrated the most knock down effect on the expression; psiU6BX-ZNFN3A1-4 which demonstrated mild silencing effect; psiU6BX-ZNFN3A1-1 which demonstrated no silencing effect, or psiU6BX-mock. MTT assays at both 6 days and 9 days of transfection showed that psiU6BX-ZNFN3A1-12 has the highest growth inhibitory effect and that psiU6BX-ZNFN3A1-1 did not change the number of surviving cells compared with cells transfected with psiU6BX-mock (Figure 4). The growth inhibitory effect of the plasmids was correlated to their gene silencing activity. To further demonstrate the growth inhibitory effect of ZNFN3A1-siRNAs, psiU6BX-ZNFN3A1-12; psiU6BX-EGFP For the control, psiU6BX-EGFP was prepared by cloning the following double-stranded oligonucleotide and
5'- AAAAGAAGCAGCACGACTTCTTCTCTCTTGAAGAAGAAGTCGTGCT GCTTC -3' (SEQ ID No: 41) into the BbsI site of the psiU6BX vector.
or psiU6BX-mock was transfected into various hepatoma cell lines including SNU398, SNU423, SNU449, Huh7, Alexander, and HepG2 and two colon cancer cell lines, SW948 and HCT116. Transfection of psiU6BX-ZNFN3A1-12 significantly reduced number of surviving cells compared with that of psiU6BX-EGFP or psiU6BX-mock (Figure 5). Furthermore, FACS analysis demonstrated that transfection of psiU6BX-ZNFN3A1-12 increased the number of cells in sub-G1 phase (Figure 6). These results indicate that ZNFN3A1 contributes to aberrant cell growth and/or survival in a wide range of human cancer cells.

### Industrial Applicability

The present inventors have shown that the cell growth is suppressed by small interfering RNA (siRNA) that specifically target the ZNFN3A1 gene. Thus, this novel siRNAs are useful target for the development of anti-cancer pharmaceuticals. For example, agents that block the expression of ZNFN3A1 or prevent its activity may find therapeutic utility as anti-cancer agents, particularly anti-cancer agents for the treatment of liver cancer or colon cancer, such as HCC or colorectal adenocarcinoma.

While the invention has been described in detail and with reference to specific embodiments thereof, it will be apparent to one skilled in the art that various changes and modifications can be made therein without departing from the scope of the invention.

### Reference

(1) Akriviadis EA, Llovet JM, Efremidis SC, Shouval D, Canelo R, Ringe B, Meyers WC. Hepatocellular carcinoma. Br J Surg. 1998 Oct;85(10):1319-31.
(2) Okabe H, Satoh S, Kato T, Kitahara O, Yanagawa R, Yamaoka Y, Tsunoda T, Furukawa Y, Nakamura Y. Genome-wide analysis of gene expression in human hepatocellular carcinomas using cDNA microarray: identification of genes involved in viral carcinogenesis and tumor progression. Cancer Res. 2001 Mar 1;61(5):2129-37.
(3) Sharp. PA. RNAi and double-strand RNA. Genes Dev. 1999 Jan 15;13(2):139-41.
(4) Hammond SM. Bernstein E, Beach D, Hannon GJ. An RNA-directed nuclease mediates post-transcriptional gene silencing in Drosophila cells. Nature. 2000 Mar 16;404(6775):293-6.
(5) Hannon GJ. RNA interference. Nature. 2002 Jul 11;418(6894):244-51.
(6) Elbashir SM, Harborth J. Lendeckel W, Yalcin A, Weber K, Tuschl T. Duplexes of 21-nucleotide RNAs mediate RNA interference in cultured mammalian cells. Nature. 2001 May 24;411(6836):494-8.
(7) Miyagishi M, Taira K. U6 promoter-driven siRNAs with four uridine 3' overhangs efficiently suppress targeted gene expression in mammalian cells.Nat Biotechnol. 2002 May;20(5):497-500
(8) Brummelkamp TR, Bernards R, Agami R. A System for Stable Expression of Short Interfering RNAs in Mammalian Cells Science. 296(5567):550-553, April 19, 2002.

## Claims

1. A composition comprising a ZNFN3A1 small interfering RNA (siRNA) for use in treating a tumor **characterized by** over-expressing ZNFN3A1 in a subject, wherein said siRNA comprises a sense ZNFN3A1 nucleic acid and an anti-sense ZNFN3A1 nucleic acid, and wherein said siRNA is specific for a ZNFN3A1 target selected from the group consisting of nucleotides 451-471, 532-552, 623-643, 625-645, 636-656,726-746, 923-943, 1065-1085, and 1258-1278 of SEQ ID NO:1, wherein said ZNFN3A1 siRNA is between 19 and 25 nucleotides in length.

2. Use of a composition comprising a ZNFN3A1 small interfering RNA (siRNA) for the preparation of a pharmaceutical composition for treating a tumor **characterized by** over-expressing ZNFN3A1 in a subject, wherein said siRNA comprises a sense ZNFN3A1 nucleic acid and an anti-sense ZNFN3A1 nucleic acid, and wherein said siRNA is specific for a ZNFN3A1 target selected from the group consisting of nucleotides 451-471, 532-552, 623-643, 625-645, 636-656,726-746, 923-943, 1065-1085, and 1258-1278 of SEQ ID NO:1, wherein said ZNFN3A1 siRNA is between 19 and 25 nucleotides in length.

3. The composition for use of claim 1 or the use of claim 2, wherein said siRNA has the general formula 5'-[A]-[B]-[A']-3', wherein [A] is a ribonucleotide sequence corresponding to a sequence selected from the group consisting of nucleotides 451-471, 532-552, 623-643, 625-645, 636-656, 726-746, 923-943, 1065-1085, and 1258-1278 of SEQ ID NO:1,
[B] is a ribonucleotide sequence consisting of 3 to 23 nucleotides, and
[A'] is a ribonucleotide sequence consisting of the complementary sequence of [A].

4. The composition for use of claim 3 or the use of claim 3, wherein said composition comprises a transfection-enhancing agent.

5. A polynucleotide for use in treating a tumor **characterized by** over-expressing ZNFN3A1, wherein the polynucleotide comprises a combination of a sense strand nucleic acid and an antisense strand nucleic acid, wherein said sense strand nucleic acid comprises a nucleotide sequence selected from the group consisting of nucleotides 451-471, 532-552, 623-643, 625-645, 636-656, 726-746, 923-943, 1065-1085, and 1258-1278 of SEQ ID NO:1, and said antisense strand nucleic acid consists of the complementary sequence of said nucleotide sequence, wherein said polynucleotide is an oligonucleotide which is between 19 and 25 nucleotides in length.

6. The polynucleotide for use of claim 5, wherein said sense strand nucleic acid and antisense strand nucleic acid are on the same strand.

7. A vector for use in treating a tumor **characterized by** over-expressing ZNFN3A1, wherein the vector comprises a polynucleotide comprising a sequence combination of a sense strand nucleic acid and an antisense strand nucleic acid, wherein said sense strand nucleic acid comprises a nucleotide sequence selected from the group consisting of nucleotides 451-471, 532-552, 623-643, 625-645, 636-656, 726-746, 923-943, 1065-1085, and 1258-1278 of SEQ ID NO:1, and said antisense strand nucleic acid consists of the complementary sequence of said nucleotide sequence, wherein said polynucleotide is an oligonucleotide which is between 19 and 25 nucleotides in length.

8. The vector of claim 7, wherein said polynucleotide has the general formula 5'-[A]-[B]-[A']-3', wherein [A] is a nucleotide sequence selected from the group consisting of nucleotides 451-471, 532-552, 623-643, 625-645, 636-656, 726-746, 923-943, 1065-1085, and 1258-1278 of SEQ ID NO:1,
[B] is a nucleotide sequence consisting of 3 to 23 nucleotides, and
[A'] is a nucleotide sequence consisting of the complementary sequence of [A].

9. A composition for use in treating a tumor **characterized by** over-expressing ZNFN3A1, wherein the composition comprises at least one siRNA comprising a combination of a sense strand nucleic acid and an antisense strand nucleic acid, wherein said sense strand nucleic acid comprises a ribonucleotide sequence corresponding to a sequence selected from the group consisting of nucleotides 451-471, 532-552, 623-643, 625-645, 636-656, 726-746, 923-943, 1065-1085, and 1258-1278 of SEQ ID NO:1, and said antisense strand sequence consists of the complementary sequence of said nucleotide sequence, wherein said siRNA is between 19 and 25 nucleotides in length.

10. A double-stranded molecule for use in treating a tumor **characterized by** over-expressing ZNFN3A1, wherein the double-stranded molecule comprises a sense strand and an antisense strand, wherein the sense strand comprises a ribonucleotide sequence corresponding to a ZNFN3A1 target sequence, and wherein the antisense strand comprises a ribonucleotide sequence which is complementary to said sense strand, wherein said sense strand and said antisense strand hybridize to each other to form said double-stranded molecule, wherein said double-stranded molecule, when introduced into a cell expressing the ZNFN3A1 gene, inhibits expression of said gene, and wherein said ZNFN3A1 target sequence is selected from the group consisting of nucleotides 451-471, 532-552, 623-643, 625-645, 636-656,726-746, 923-943, 1065-1085, and 1258-1278 of SEQ ID NO:1, wherein said double-stranded molecule is an oligonucleotide of between 19 and 25 nucleotides in length.

11. The double-stranded molecule for use of claim 10, wherein a single ribonucleotide transcript comprises the sense strand and the antisense strand, said double-stranded molecule further comprising a single-stranded ribonucleotide sequence linking said sense strand and said antisense strand.

12. A vector encoding the double-stranded molecule of claim 10 or 11 for use of claim 10.

13. The vector for use of claim 12, wherein the vector encodes a transcript having a secondary structure, wherein the transcript comprises the sense strand and the antisense strand.

14. The vector for use of claim 13, wherein the transcript further comprises a single-stranded ribonucleotide sequence linking said sense strand and said antisense strand.

15. The composition for use of claim 1 or 9, the use of claim 2, the polynucleotide for use of claim 5, the vector for use of claim 7 or the double-stranded molecule for use of claim 10, wherein said tumor is a colorectal cancer or liver cancer.

16. The composition for use, the use, the polynucleotide for use, the vector for use or the double-stranded molecule for use of claim 15, wherein
(a) the colorectal cancer is an adenocarcinoma; or
(b) the liver cancer is a hepatocellular carcinoma.

## Patentansprüche

1. Zusammensetzung, umfassend eine ZNFN3A1-kurze interferierende RNA (siRNA) zur Behandlung eines Tumors, welcher durch ZNFN3A1-Überexpression charakterisiert ist, in einem Individuum, wobei die siRNA eine Sense-ZNFN3A1-Nucleinsäure und eine Antisense-ZNFN3A1-Nucleinsäure umfasst, und wobei die siRNA spezifisch für ein ZNFN3A1-Ziel ist, welches ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1, wobei die ZNFN3A1-siRNA zwischen 19 und 25 Nucleotide lang ist.

2. Verwendung einer Zusammensetzung umfassend eine ZNFN3A1-kurze interferierende RNA (siRNA) für die Herstellung eines Arzneimittels zur Behandlung eines Tumors, welcher durch ZNFN3A1-Überexpression charakterisiert ist, in einem Individuum, wobei die siRNA eine Sense-ZNFN3A1-Nucleinsäure und eine Antisense-ZNFN3A1-Nucleinsäure umfasst, und wobei die siRNA spezifisch ist für ein ZNFN3A1-Ziel, welches ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1, wobei die ZNFN3A1-siRNA zwischen 19 und 25 Nucleotide lang ist.

3. Zusammensetzung zur Verwendung nach Anspruch 1 oder Verwendung nach Anspruch 2, wobei die siRNA die allgemeine Formel 5'-[A]-[B]-[A']-3' hat, wobei [A] eine Ribonucleotidsequenz ist, die einer Sequenz entspricht, welche ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1,
[B] eine Ribonucleotidsequenz ist, die aus 3 bis 23 Nucleotiden besteht, und
[A'] eine Ribonucleotidsequenz ist, welche aus der komplementären Sequenz zu [A] besteht.

4. Zusammensetzung zur Verwendung nach Anspruch 3 oder Verwendung nach Anspruch 3, wobei die Zusammensetzung ein Transfektions-verstärkendes Mittel umfasst.

5. Polynucleotid zur Verwendung in der Behandlung eines Tumors, welcher durch ZNFN3A1-Überexpression charakterisiert ist, wobei das Polynucleotid eine Kombination eines Sense-Nucleinsäurestrangs und eines Antisense-Nucleinsäurestrangs umfasst, wobei der Sense-Nucleinsäurestrang eine Nucleotidsequenz umfasst, welche ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1, und der Antisense-Nucleinsäurestrang aus der komplementären Sequenz zu der Nucleotidsequenz besteht, wobei das Polynucleotid ein Oligonucleotid ist, das zwischen 19 und 25 Nucleotide lang ist.

6. Polynucleotid zur Verwendung nach Anspruch 5, wobei der Sense-Nucleinsäurestrang und der Antisense-Nucleinsäurestrang auf demselben Strang sind.

7. Vektor zur Verwendung in der Behandlung eines Tumors, welcher durch ZNFN3A1-Überexpression charakterisiert ist, wobei der Vektor ein Polynucleotid umfasst, welches eine Sequenzkombination eines Sense-Nucleinsäurestrangs und eines Antisense-Nucleinsäurestrangs umfasst, wobei der Sense-Nucleinsäurestrang eine Nucleotidsequenz umfasst, welche ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1, und der Antisense-Nucleinsäurestrang aus der komplementären Sequenz zu der Nucleotidsequenz besteht, wobei das Polynucleotid ein Oligonucleotid ist, das zwischen 19 und 25 Nucleotide lang ist.

8. Vektor zur Verwendung nach Anspruch 7, wobei das Polynucleotid die allgemeine Formel 5'-[A]-[B]-[A']-3' hat, wobei [A] eine Nucleotidsequenz ist, welche ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1,
[B] eine Nucleotisequenz ist, die aus 3 bis 23 Nucleotiden besteht, und
[A'] eine Nucleotidsequenz ist, welche aus der komplementären Sequenz zu [A] besteht.

9. Zusammensetzung zur Verwendung in der Behandlung eines Tumors, welcher durch ZNFN3A1-Überexpression charakterisiert ist, wobei die Zusammensetzung mindestens eine siRNA umfasst, die eine Kombination eines Sense-Nucleinsäurestrangs und eines Antisense-Nucleinsäurestrangs umfasst, wobei der Sense-Nucleinsäurestrang eine Ribonucleotidsequenz umfasst, die einer Sequenz entspricht, welche ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1, und der Antisense-Strang aus der komplementären Sequenz zu der Nucleotidsequenz besteht, wobei die siRNA zwischen 19 und 25 Nucleotide lang ist.

10. Doppelsträngiges Molekül zur Verwendung in der Behandlung eines Tumors, welcher durch ZNFN3A1-Überexpression charakterisiert ist, wobei das doppelsträngige Molekül einen Sense-Strang und einen Antisense-Strang umfasst, wobei der Sense-Strang eine Ribonucleotidsequenz umfasst, die einer ZNFN3A1-Zielsequenz entspricht, und wobei der Antisense-Strang eine Ribonucleotidsequenz umfasst, welche komplementär zu dem Sense-Strang ist, wobei der Sense-Strang und der Antisense-Strang miteinander hybridisieren unter Bildung des doppelsträngigen Moleküls, wobei das doppelsträngige Molekül, wenn es in eine Zelle, welche das ZNFN3A1-Gen exprimiert, eingeführt wird, die Expression des Gens hemmt, und wobei die ZNFN3A1-Zielsequenz ausgewählt ist aus der Gruppe bestehend aus den Nucleotiden 451-471, 532-552, 623-643, 625-645, 636-656, 726-746, 923-943, 1065-1085 und 1258-1278 der SEQ ID NO: 1; wobei das doppelsträngige Molekül ein Oligonucleotid mit einer Länge zwischen 19 und 25 Nucleotiden ist.

11. Doppelsträngiges Molekül zur Verwendung nach Anspruch 10, wobei ein einzelnes Ribonucleotid-Transkript den Sense-Strang und den Antisense-Strang umfasst, wobei das doppelsträngige Molekül ferner eine einzelsträngige Ribonucleotidsequenz umfasst, welche den Sense-Strang und den Antisense-Strang verbindet.

12. Vektor, welcher das doppelsträngige Molekül nach Anspruch 10 oder 11 codiert, zur Verwendung nach Anspruch 10.

13. Vektor zur Verwendung nach Anspruch 12, wobei der Vektor ein Transkript codiert, das eine Sekundärstruktur hat, wobei das Transkript den Sense-Strang und den Antisense-Strang umfasst.

14. Vektor zur Verwendung nach Anspruch 13, wobei das Transkript ferner eine einzelsträngige Ribonucleotidsequenz umfasst, welche den Sense-Strang und den Antisense-Strang verbindet.

15. Zusammensetzung zur Verwendung nach Anspruch 1 oder 9, Verwendung nach Anspruch 2, Polynucleotid zur Verwendung nach Anspruch 5, Vektor zur Verwendung nach Anspruch 7 oder doppelsträngiges Molekül zur Verwendung nach Anspruch 10, wobei der Tumor ein Kolorektalkrebs oder Leberkrebs ist.

16. Zusammensetzung zur Verwendung, Verwendung, Polynucleotid zur Verwendung, Vektor zur Verwendung oder doppelsträngiges Molekül zur Verwendung nach Anspruch 15, wobei
(a) der Kolorektalkrebs ein Adenokarzinom; oder
(b) der Leberkrebs ein hepatozelluläres Karzinom ist.

## Revendications

1. Composition comprenant un petit ARN interférent (ARNsi) de ZNFN3A1, destinée à être utilisée dans le traitement d'une tumeur **caractérisée par** une surexpression de ZNFN3A1 chez un sujet, dans laquelle ledit ARNsi comprend un acide nucléique de ZNFN3A1 sens et un acide nucléique de ZNFN3A1 antisens, et dans laquelle ledit ARNsi est spécifique pour une cible de ZNFN3A1 sélectionnée dans le groupe consistant en les nucléotides 451-471, 532-552, 623-643, 625-645, 636-656, 726-746, 923-943, 1065-1085, et 1258-1278 de SEQ ID NO: 1, où ledit ARNsi de ZNFN3A1 a une longueur comprise entre 19 et 25 nucléotides.

2. Utilisation d'une composition comprenant un petit ARN interférent (ARNsi) de ZNFN3A1 pour la préparation d'une composition pharmaceutique destinée au traitement d'une tumeur **caractérisée par** une surexpression de ZNFN3A1 chez un sujet, où ledit ARNsi comprend un acide nucléique de ZNFN3A1 sens et un acide nucléique de ZNFN3A1 antisens, et où ledit ARNsi est spécifique pour une cible de ZNFN3A1 sélectionnée dans le groupe consistant en les nucléotides 451-471, 532-552, 623-643, 625-645, 636-656, 726-746, 923-943, 1065-1085, et 1258-1278 de SEQ ID NO: 1, où ledit ARNsi de ZNFN3A1 a une longueur comprise entre 19 et 25 nucléotides.

3. Composition destinée à une utilisation selon la revendication 1 ou la revendication 2, dans laquelle ledit ARNsi possède la formule générale 5'-[A]-[B]-[A']-3', où [A] est une séquence ribonucléotidique correspondant à une séquence sélectionnée dans le groupe consistant en les nucléotides 451-471, 532-552, 623-643, 625-645, 636-656, 726-746, 923-943, 1065-1085, et 1258-1278 de SEQ ID NO: 1,
[B] est une séquence ribonucléotidique consistant en 3 à 23 nucléotides, et
[A'] est une séquence ribonucléotidique consistant en la séquence complémentaire de [A].

4. Composition destinée à une utilisation selon la revendication 3, où ladite composition comprend un agent améliorant la transfection.

5. Polynucléotide destiné à être utilisé dans le traitement d'une tumeur **caractérisée par** une surexpression de ZNFN3A1, où le polynucléotide comprend une combinaison d'un acide nucléique de brin sens et d'un acide nucléique de brin antisens, où ledit acide nucléique de brin sens comprend une séquence de nucléotides sélectionnée dans le groupe consistant en les nucléotides 451-471, 532-552, 623-643, 625-645, 636-656, 726-746, 923-943, 1065-1085, et 1258-1278 de SEQ ID NO: 1, et ledit acide nucléique de brin antisens consiste en la séquence complémentaire de ladite séquence de nucléotides, où ledit polynucléotide est un oligonucléotide ayant une longueur comprise entre 19 et 25 nucléotides.

6. Polynucléotide destiné à une utilisation selon la revendication 5, dans lequel les dits acides nucléiques du brin sens et acide nucléique de brin antisens sont sur le même brin.

7. Vecteur destiné à être utilisé dans le traitement d'une tumeur **caractérisée par** une surexpression de ZNFN3A1, où le vecteur comprend un polynucléotide comprenant une combinaison de séquences d'un acide nucléique de brin sens et d'un acide nucléique de brin antisens, où ledit acide nucléique de brin sens comprend une séquence de nucléotides sélectionnée dans le groupe consistant en les nucléotides 451-471, 532-552, 623-643, 625-645, 636-656, 726-746, 923-943, 1065-1085, et 1258-1278 de SEQ ID NO: 1, et ledit acide nucléique de brin antisens consiste en la séquence complémentaire de ladite séquence de nucléotides, où ledit polynucléotide est un oligonucléotide ayant une longueur comprise entre 19 et 25 nucléotides.

8. Vecteur destiné à une utilisation selon la revendication 7, où ledit polynucléotide possède la formule générale 5'-[A]-[B]-[A']-3', où [A] est une séquence de nucléotides sélectionnée dans le groupe consistant en les nucléotides 451-471, 532-552, 623-643, 625-645, 636-656, 726-746, 923-943, 1065-1085, et 1258-1278 de SEQ ID NO: 1,
[B] est une séquence de nucléotides consistant en 3 à 23 nucléotides, et
[A'] est une séquence de nucléotides consistant en la séquence complémentaire de [A].

9. Composition destinée à une utilisation dans le traitement d'une tumeur **caractérisée par** une surexpression de ZNFN3A1, où la composition comprend au moins un ARNsi comprenant une combinaison d'un acide nucléique de brin sens et d'un acide nucléique de brin antisens, où ledit acide nucléique de brin sens comprend une séquence ribonucléotidique correspondant à une séquence sélectionnée dans le groupe consistant en les nucléotides 451-471, 532-552, 623-643, 625-645, 636-656, 726-746, 923-943, 1065-1085, et 1258-1278 de SEQ ID NO: 1, et ladite séquence de brin antisens consiste en la séquence complémentaire de ladite séquence de nucléotides, où ledit ARNsi a une longueur comprise entre 19 et 25 nucléotides.

10. Molécule double brin destinée à une utilisation dans le traitement d'une tumeur **caractérisée par** une surexpression de ZNFN3A1, où la molécule double brin comprend un brin sens et un brin antisens, où le brin sens comprend une séquence ribonucléotidique correspondant à une séquence cible de ZNFN3A1, et où le brin antisens comprend une séquence ribonucléotidique qui est complémentaire audit brin sens, où ledit brin sens et ledit brin antisens s'hybrident l'un à l'autre pour former ladite molécule double brin, où ladite molécule double brin, lorsqu'elle est introduite dans une cellule exprimant le gène ZNFN3A1, inhibe l'expression dudit gène, et où ladite séquence cible de ZNFN3A1 est sélectionnée dans le groupe consistant en les nucléotides 451-471, 532-552, 623-643, 625-645, 636-656, 726-746, 923-943, 1065-1085, et 1258-1278 de SEQ ID NO: 1, où ladite molécule double brin est un oligonucléotide ayant une longueur comprise entre 19 et 25 nucléotides.

11. Molécule double brin destinée à une utilisation selon la revendication 10, dans laquelle un seul transcrit de ribonucléotide comprend le brin sens et le brin antisens, ladite molécule double brin comprenant en outre une séquence ribonucléotidique simple brin liant ledit brin sens et ledit brin antisens.

12. Vecteur codant pour la molécule double brin selon la revendication 10 ou 11, destinée à une utilisation selon la revendication 10.

13. Vecteur destiné à une utilisation selon la revendication 12, où le vecteur code pour un transcrit ayant une structure secondaire, où le transcrit comprend le brin sens et le brin antisens.

14. Vecteur destiné à une utilisation selon la revendication 13, où le transcrit comprend en outre une séquence ribonucléotidique simple brin liant ledit brin sens et ledit brin antisens.

15. Composition destinée à une utilisation selon la revendication 1 ou 9, utilisation selon la revendication 2, polynucléotide destiné à une utilisation selon la revendication 5, vecteur destiné à une utilisation selon la revendication 7 ou molécule double brin destinée à une utilisation selon la revendication 10, où ladite tumeur est un cancer colorectal ou un cancer du foie.

16. Composition destinée à une utilisation, utilisation, polynucléotide destiné à une utilisation, vecteur destiné à une utilisation ou molécule double brin destinée à une utilisation selon la revendication 15, où
(a) le cancer colorectal est un adénocarcinome; ou
(b) le cancer du foie est un carcinome hépatocellulaire.
